(19) Europäisches Patentamt
European Patent Office
Office européen des brevets

(11) **EP 1 961 850 B1**

(12) **EUROPEAN PATENT SPECIFICATION**

(45) Date of publication and mention
of the grant of the patent:
**28.10.2009 Bulletin 2009/44**

(51) Int Cl.:
***D04H 13/00*** (2006.01)   ***D04H 3/16*** (2006.01)
***D04H 3/10*** (2006.01)   ***D04H 1/46*** (2006.01)

(21) Application number: **07003616.5**

(22) Date of filing: **22.02.2007**

(54) **HYDROENTANGLED COMPOSITE NONWOVEN COMPRISING A SPUNBONDED LAYER AND AN ABSORBENT PULP LAYER, METHOD AND CONTINUOUS SYSTEM FOR PRODUCING SAID COMPOSITE**

WASSERSTRAHLVERFESTIGTER VERBUNDVLIES MIT SPINNVLIESSCHICHT UND SAUGFÄHIGER ZELLSTOFFSCHICHT, VERFAHREN UND KONTINUIERLICHES SYSTEM ZUR HERSTELLUNG DIESES VERBUNDSTOFFES

COMPOSITE NON TISSÉ ENCHEVÊTRÉ PAR VOIE HYDRAULIQUE COMPRENANT UNE COUCHE PAR FILAGE DIRECT ET UNE COUCHE DE PÂTE ABSORBANTE, PROCÉDÉ DE PRODUCTION DE CE COMPOSITE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**27.08.2008 Bulletin 2008/35**

(73) Proprietor: **ALBIS Spa**
**20123 Milano (IT)**

(72) Inventor: **Boscolo, Galliano**
**13856 Vigliano B.SE (IT)**

(74) Representative: **Matkowska, Franck**
**Matkowska & Associés**
**9 Rue Jacques Prévert**
**59650 Villeneuve d'Ascq (FR)**

(56) References cited:
**WO-A-01/53590       WO-A-2004/092472**
**WO-A-2006/010766    WO-A-2006/092814**
**US-A- 3 692 618      US-A- 4 340 563**
**US-A- 5 284 703      US-A1- 2004 121 693**
**US-B2- 6 836 938**

## Description

### Field of the Invention

The present invention relates to a novel hydroentangled composite nonwoven for absorbing liquids and comprising at least a pre-consolidated spunbonded layer and an absorbent pulp layer. The invention also relates to a method and continuous system for producing said novel absorbent composite nonwoven.

### Prior art

Absorbent composite nonwoven are widely used in the prior art for absorbing liquids, especially, but not only, in the hygienic industry for making products such as, for example, diapers or sanitary napkins.

Absorbent composite nonwovens generally comprise at least two layers: a consolidated nonwoven carrier and an absorbent layer.

An absorbent material widely used for making the absorbent layer is a fibre material generally referred as "pulp", and made of or containing fibres from natural sources such as woody and non-woody plants. Woody plants include, for example, deciduous and coniferous trees. Non-woody plants include, for example, cotton, flax, esparto grass, milkweed, straw, jute hemp, and bagasse.

In European patent application EP 0 540 041, a process for making a hydroentangled absorbent composite nonwoven is being disclosed. Said process comprises the steps of:

- providing a nonwoven carrier,
- hydraulically needling the nonwoven carrier, in order mainly to enhance the liquid distribution properties of the nonwoven carrier,
- applying and bonding a layer of absorbent material, including pulp, onto the surface of the nonwoven carrier.

It has become apparent that pure consolidation of the composite absorbent nonwoven by compression only produces an insufficiently secure contact between the absorbent layer and the carrier nonwoven.

A satisfactory connection of an absorbent pulp layer to a nonwoven carrier is known, e.g. from US patent No 3,560,328 or PCT application WO92/080834, specifically through hydraulic needling of the pulp fibres of the absorbent layer with the consolidated nonwoven carrier.

As pointed out in US patent No 6, 836, 937, the hydraulic needling of the pulp fibres of the absorbent layer with the consolidated nonwoven carrier results however in a high loss of pulp fibres. According to US patent No 6, 836, 937, tests have shown that up to 12% of the pulp fibres are washed out of the useful absorbent layer or bond, and are thus lost for the efficiency of the product. Moreover, in theses processes, very many lost pulp fibres get into the filtration means that are necessary for treating and recycling water, in case of water needling. This increases the costs for the water recycling process, and thereby the manufacturing costs of the absorbent composite nonwoven.

US patent No 6, 836, 937 discloses a new process that overcomes this problem of high pulp loss. This process consists essentially in inserting a thin intermediate meltblown layer between the nonwoven carrier and the absorbent pulp layer- This technical solution increases however the production costs, since it involves the manufacture of a supplementary layer between the nonwoven carrier and the absorbent pulp layer.

PCT applications WO 2004/092472 and WO 2006/010766 disclose a method for manufacturing a hydroentangled composite nonwoven comprising a spunbonded layer and a pulp layer. More particularly, it is recommended in these publications to spin splittable multicomponent polymers filaments for making the spunbonded layer. These splittable multicomponent polymers filaments are composed of microfilaments having a count between 0.1dtex and 0.9dtex, and the splittable filaments have a count between 1.7dtex and 2.2dtex. The splitting of the filaments is obtained during the hydroentanglement step of the spunbonded layer.

PCT application WO 01/53588 and US patent No 6,836,938 disclose a method for producing a composite nonwoven, in particular for the production of a hygienic product, said method comprising the following steps:

- forming a spunbonded nonwoven layer,
- compressing and optionally thermo-bonding the spunbonding layer in order to obtain a light bonding of the fibres of the spunbonded layer,
- coating the pre-consolidated spunbonded layer with a layer of pulp fibres,
- conducting a hydrodynamic water needling process, in order to interconnect and strengthen the layer of pulp fibres and the pre-consolidated spunbonded layer.

One objective of this publication is to reduce the pulp loss during hydrodynamic water needling, and for achieving this objective, it is recommended in this publication to make only a light bonding of the fibres of the spunbonded layer during the compaction and optional thermo-bonding step, in such a way that the pulp fibres enter into an internal bonding with the fibres of the spunbonded nonwoven fabric in the hydrodynamic water needling.

Publication US 2003/0207636 and PCT application WO01/53590 disclose a hydroentangled and absorbent composite nonwoven comprising a bulk pulp layer and a spunbonded layer made of fine denier filaments, typically in the range of 0.5 denier and 1.2 denier (i.e. 0.55 dtex and 1.3 dtex). In this publication, it is pointed out that the type of bonding of the spunbonded layer is not believed to be critical and may include, for example, solvent adhesive, needling, hydroentanglement, or thermal bonding.

## Objectives of the invention

An objective of the invention is to propose a novel hydroentangled absorbent composite nonwoven comprising at least a spunbonded layer and an absorbent pulp layer for absorbing liquids, and a process and continuous system for producing said novel composite nonwoven.

Another objective of the invention is to obtain a hydroentangled absorbent composite nonwoven exhibiting improved mechanical properties, in particular good tensile properties, and good abrasion resistance properties.

Another objective of the invention is to obtain a hydroentangled absorbent composite nonwoven exhibiting an improved softness.

Another objective of the invention is to reduce pulp losses during the manufacturing process of the composite, especially during the hydrodynamic water needling step of the layers of the composite.

## Summary of the invention

The novel method of the invention for producing a hydroentangled absorbent composite nonwoven is defined in claim 1. Said method comprises the steps of:

(a) forming one spunbonded layer (A'),
(b) thermo-bonding the said spunbonded layer (A'), in order to obtain a pre-consolidated spunbonded layer (A),
(c) laying one absorbent pulp layer (B) onto and in contact with the pre-consolidated spunbonded layer (A),
(d) consolidating the composite nonwoven by hydrodynamic needling.

According to the invention, the spunbonded layer (A') formed in step (a) comprises continuous microfilaments having a diameter (DI) less than or equal to $15 \mu m$, and the pre-consolidation step (b) of the spunbonded layer (A') is performed by means of a bonding pattern having bonding dots, the density of said bonding dots being higher than or equal to 90 dots/cm$^2$.

The said spunbonded layer, that comprises very fine continuous microfilaments ($DI \leq 15 \mu m$) and is pre-consolidated with a microbonding pattern having a very high bonding dots density ($\geq 90$ dots/cm$^2$), advantageously constitutes a good barrier for the pulp fibres during the hydrodynamic needling of the composite, thereby reducing the pulp loss, and enables to achieve good mechanical properties, good uniformity, and good softness for the composite nonwoven.

Preferably, but not necessarily, all steps (a), (b), (c) and (d) of the method of the invention are advantageously performed continuously on one production line. But within the scope of the invention, some steps of the method can be performed separately on separate production lines. For example, the pre-consolidated spunbonded layer (A) can be produced on a first production line (steps (a) and (b)), and stored in the form of a roll. Then this pre-consolidated spunbonded layer (A) is transported to a second production line, where it can be used for performing the following steps (c) and (d) of the method of the invention. In a similar way, when the method of the invention comprises an additional step of providing a nonwoven cover layer (C) onto and in contact with the absorbent pulp layer (B) before the step (d) of consolidating the composite nonwoven, the said nonwoven cover (C) can be either produced continuously in line with the other steps on the same production line, or can be produced separately on a first production line, and be transported and used on a second production line where the other steps are performed.

The terms "pulp layer" used therein and in the claims encompass any absorbent layer essentially comprising pulp.

The term "pulp" as used therein and in the claims refers to absorbent material made of or containing fibres from natural sources such a as woody and non-woody plants. Woody plants include, for example, deciduous and coniferous trees. Non-woody plants include, for example, cotton, flax, esparto grass, milkweed, straw, jute hemp, and bagasse.

Within the scope of the invention, the absorbent pulp layer can be made solely of pulp fibres, but can be also be made of a dry mixture of pulp fibres with other materials, provided the said mixture can be dry-laid onto the consolidated spunbonded layer of the invention, by air-laid techniques or the like.

In a particular variant, the method of the invention further comprises an additional step of providing a nonwoven cover layer (C) onto and in contact with the absorbent pulp layer (B) before the step (d) of consolidating the composite nonwoven. More preferably, this additional step comprises the following sub-steps:

(a') forming one spunbonded layer (C')
(b') thermo-bonding the said spunbonded layer (C'), in order to obtain a pre-consolidated spunbonded layer (C).

The present invention further relates to a novel hydroentangled absorbent composite nonwoven defined in independent claim 20.

The present invention further relates to a novel continuous system defined in independent claim 38, for producing the composite nonwoven of the invention.

The hydroentangled absorbent composite nonwoven of the invention can be used advantageously in all applications, where the absorption of liquid is needed.

The hydroentangled absorbent composite nonwoven of the invention is preferably used, but not only, in the field of hygienic industry, for making absorbent hygienic products. The present invention thus further rotates to the use of this novel composite nonwoven for making hygienic products, and more particularly dry wipes, or wet wipes, or diapers, or training pants, or sanitary napkins, or incontinence products.

Additional and optional characteristics of the invention are also defined in the attached claims.

**Brief description of the drawings**

The characteristics and advantages of the invention will appear more clearly on reading the following detailed description which is made by way of non-exhaustive and non-limiting example, and with reference to the accompanying drawings, in which:

- Figure 1 is a general and schematic drawing of an example of absorbent composite nonwoven of the invention,
- Figure 2 is a schematic drawing of a first continuous system for producing the composite nonwoven of figure 1, and
- Figure 3 is a schematic drawing of a second continuous system for producing the composite nonwoven of figure 1,
- Figure 4 is a schematic drawing of a third continuous system for producing the composite nonwoven of figure 1,
- Figure 5 is plane view of an example of micro-bonding pattern that is suitable for practising the invention and that is referred as C#1 in the following detailed description,
- Figure 6 is a view in cross section of the micro-bonding pattern of figure 5, in plane VI-VI of figure 5,
- Figure 7 is a view in cross section of the micro-bonding pattern of figure 5, in plane VII-VII of figure 5,
- Figure 8 is plane view of an example of an other bonding pattern that is referred as C#2 in the following detailed description,
- Figure 9 is a view in cross section of the bonding pattern of figure 8, in plane IX-IX of figure 8,
- Figure 10 is a photography of a sample of pre-consolidated spunbonded web (A) of the invention taken with a microscope,
- Figure 11 is a photography of a sample of composite nonwoven of the invention (Spunbonded/Pulp/Carded) described hereafter and taken with a microscope on the spunbonded side (A) of the composite,
- Figures 12A to 12H are different examples of spun filaments cross-sections that are suitable for practising the invention.

**Detailed description**

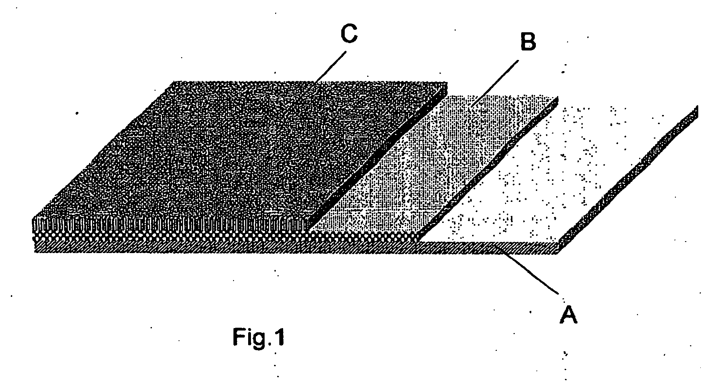

Referring to figure 1, a hydroentangled absorbent composite nonwoven of the invention comprises at least two superposed layers : a pre-consolidated spunbonded web A, and an absorbent pulp layer B, that is adjacent to said pre-consolidated spunbonded web A. In the preferred embodiment of figure 1, the composite nonwoven optionally comprises a nonwoven cover layer C adjacent to the absorbent pulp layer B, said pulp layer B being sandwiched between the spunbonded web A and the nonwoven cover layer C.

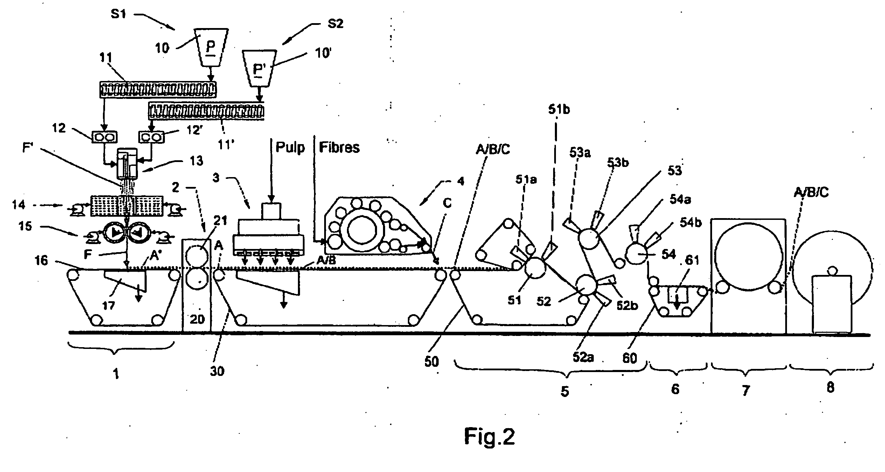

The composite nonwoven (A/B/C) of figure 1 is, for example, advantageously manufactured by means of the continuous system of figure 2.

The continuous system of figure 2 comprises a spunbonding unit 1, a thermal bonding unit 2, an air-laid unit 3, a carding unit 4, a hydraulic needling unit 5, a dewatering unit 6, a drying unit 7, and a winding unit 8.

Spunbonding unit 1

The spunbonding unit 1 is used for producing a non-consolidated spunbonded web A' made of continuous spun filaments F.

The spunbonding unit 1 comprises at least one supplying line S1. Said supplying line S1 comprises a feeding hopper 10, an extruder 11 and metering pumps 12. The feeding hopper 10 contains a polymeric material P (for example in the form of pellets, or chips, or granulates,...). Said hopper 10 is connected to the inlet of the extruder 11, that enables to continuously heat up and molten the polymeric material P. The outlet of the extruder 11 is connected to the inlet of metering pumps 12, via a distribution manifold. The outlets of the metering pumps 12 are connected to the inlet of a spinning pack 13. The metering pumps 12 are used for continuously dosing the molten polymer P into the spinning pack 13. This spinning pack 13 is used for producing a curtain of continuous filaments F'.

In the particular example of figure 1, the spunbonding unit 1 further comprises a second supplying line S2 for feeding the spinning pack 13 with a polymeric material P'. This second supplying line S2 comprises a feeding hopper 10', that contains the polymeric material P', an extruder 11', and metering pumps 12'.

Downstream the spinning pack 13, the spunbonding unit 1 comprises an air quenching box 14 that is being used to cool down the filaments F' issued from the spinning pack 13, and an air drawing equipment 15 that is being used to reduce the diameter of the filaments in order to form a curtain of filaments F having a smaller diameter.

The polymeric material(s) [P and/or P'] used for making the continuous spun filaments F can be any known spinnable polymeric material, and for example, polyolefin (in particular polypropylene or polyethylene), polyester, or polyamide, or any biodegradable thermoplastic polymer, like for example polylactic acid (PLA), or any blend thereof, or any copolymers thereof, or any blend of copolymers thereof.

These continuous spun filaments F can be, for example, monocomponent or multicomponent filaments, especially bicomponent filaments, and more especially sheath/core bicomponent filaments. When monocomponent spun filaments F are produced, only one supplying line (S1 or S2) can be used or both supplying lines can be used (S1 and S2). When bicomponent spun filaments F are produced, both supplying lines S1 and S2 are used simultaneously. In case of sheath/core bicomponent filaments, it is preferred, but not mandatory, to use polyethylene/polypropylene filaments.

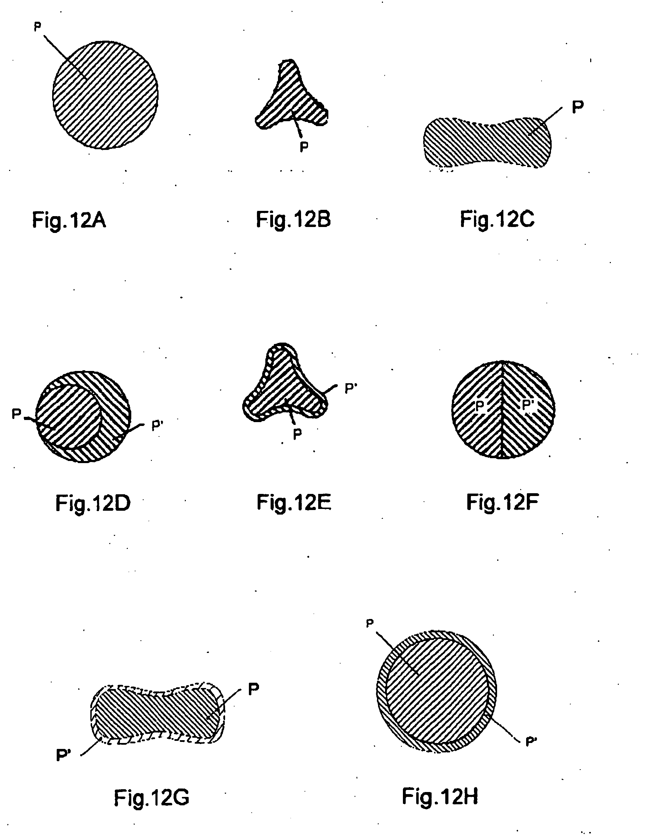

Various shapes in cross section for the filaments F can also be envisaged (round shape, oval shape, bilobal shape, trilobal shape, etc...). The shape in cross section of the continuous spun filament F is determined by the geometry of the holes of the spinneret plate of the spinning pack 13. Some non-limiting examples of different cross sections for monocomponent filaments that are suitable for the invention are illustrated on figures 12A, 12B and 12C and some non-limiting examples of different cross sections for bi-compdnent filaments that are suitable for the invention are illustrated on figures 12D, 12E, 12F, 12G, 12H.

The air drawing equipment 15 is mounted above a movable and foraminous surface, such as a wire conveyor belt 16. The spun filaments F of reduced diameter, that are issued from the air drawing equipment 15, are laid down onto the said movable surface 16, where vacuum is applied, opposite to the filaments lay down side, by means of a vacuum box 17. A non-consolidated spunbonded web A' made of continuous spun filaments F is thus formed on the surface of the belt 16, and is transported by the belt 16 towards the thermal bonding unit 2, that is mounted downstream the spunbonding unit 1.

According to a main characteristic of the invention, at least part of these continuous spun filaments F, preferably more than 50% of these continuous filaments F, and more preferably all these continuous filaments F, are microfilaments having a diameter DI less than or equal to 15μm, and more preferably less than 10μm.

Preferably, but not necessarily, the spunbonded web A' is a light web whose weight is between 7 g/m$^2$ and 35g/m$^2$, preferably less than 25 g/m$^2$ , more preferably less than 12 g/m$^2$.

The spunbonding unit 1 is knowingly set up by one skilled in the art, in order to produce such a light spunbonded web A' made of continuous spun filaments F, that comprise or are constituted by very fine spun microfilaments having a diameter DI less than or equal to 15μm, and more preferably less than or equal to 10μm.

Diameter of a filament

Whatever the shape in cross section of a filament F is, the diameter DI of said filament F can be checked as follows. A sample of a filament F is being collected (for example on the belt 16), and the filament count CT is measured by applying the following known gravimetric method: the length of the sample is measured and the sample is being weighted. The weight of the sample, expressed in g (grams), is then correlated to the weight of 10000 meters of filament, in order to obtain the filament count (in dtex). The density d of the polymeric material being known, the diameter DI (in μm) of the continuous filament F is then calculated by using the following equation (1).

$$(1) \quad DI = \sqrt{\frac{400.CT}{\pi.d}}$$

Wherein

- CT is the filament count in dtex:
- d is the density (in g/cm$^3$ or Kg/dm$^3$) of the polymeric material

In case of a monocomponent filament, the density d of the polymer is well-known in the art. By way of examples only, the density of several homopolymer that are suitable for the invention are the following:

Polypropylene (PP) : d = 0.91g/cm$^3$
Polyethylene (PE) : d = 0.95g/cm$^3$
Polyethylene terephtalate (PET) : d= 1.37g/cm$^3$
Polylactic acid (PLA) : d= 1.25g/cm$^3$

In case for example of bicomponent filaments made of two different polymers P1 and P2, having known densities d1 and d2, the density d can be calculated with the following formula :

$$(2) \quad d = \frac{K1}{K1+K2} \times d1 + \frac{K2}{K1+K2} \times d2$$

Wherein:

- K1 (is the mass flow of polymer P1 (expressed for example in Kg/h) measured by the dosing system installed between the hopper (10 or 10') and the extruder (11 or 11')
- K2 (is the mass flow of polymer P2 (expressed for example in Kg/h) measured by the dosing system installed between the hopper (10 or 10') and the extruder (11 or 11')

In case of filaments having a round shape in cross section (monocomponent or multicomponent filaments), the diameter DI of the filament can also be measured by using an optical or electronic microscope. In that case, depending on the fiber diameter uniformity, it is recommended to perform several measurements of the filament diameter at different locations along the sample length, and to calculate an average value for the diameter DI.

Thermal bonding unit 2

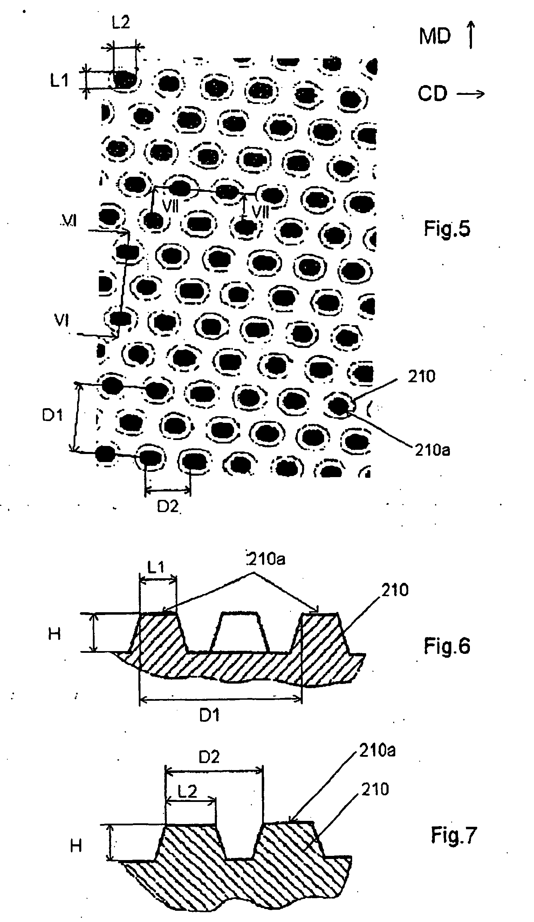

Referring to figure 2, the spunbonded web A' is fed to a thermal bonding unit 2, that is being used in order to pre-consolidate the spunbonded web A' by heat and mechanical compression (thermo-bonding), and form the pre-consolidated spunbonded web A of the composite nonwoven of figure 1.
In the particular example of figure 2, said thermal bonding unit 2 is a calender that comprises two heated pressure rolls 20, 21. The lower roll has a smooth surface, and is for example a smooth steel roll. The upper roll 21 has an engraved surface with protruding ribs, that are regularly distributed over the whole surface of the roll, and that form a micro-bonding pattern.
An example of a micro-bonding pattern for roll 21 that is suitable for practicing the invention is shown on figures 5 to 7. This micro-bonding pattern will be referred hereafter "C#1".The upper surface 210a of each protruding rib 210 forms one bonding dot.
The heating temperature of said rolls 20, 21 is set up in order to obtain a softening of the surface of the filaments F. The mechanical pressure exerted by the rolls on the spunbonded web is sufficient in order to obtain a thermo-bonding of the spun filaments F, under heat and pressure.
According to a main characteristic of the invention, the density of the bonding dots 210a (i.e. the number of bonding dots 210a per cm$^2$) of the upper engraved roll 21 is very high, and at least equal to 90 bonding dots/cm$^2$, and more preferably at least equal to 100 bonding dots/cm$^2$ ; the bonding ratio is low, and preferably less than 30% and more preferably less than 20%. The bonding ratio R is given by the following formula:

$$(3) \quad R = DD \times DA \times 100$$

Wherein:

- DD is the density of bonding dots 210a (dots/cm$^2$)
- DA A is the area of one bonding dot 210 (cm$^2$)

More particularly, the area DA of each bonding dot 210a is less than 0.5mm$^2$, preferably less than 0.3mm$^2$, and more preferably less than 0,2mm$^2$.

In the particular example of figures 5 to 7, the bonding dots 210a of the bonding pattern "C#1" have the same oval shape and the main dimensions of said bonding pattern are the followings :

Density of bonding dots (DD):102 dots/cm$^2$
Dot area (DA): 0.181mm$^2$
Bonding ratio (R): 18.5%
Dot length L1 (Machine Direction): 0.40mm
Dot width L2 (Cross Direction): 0.54mm
Dot height (H): 0.40mm
Distance (D1) between two adjacent dots in Machine Direction (MD):1.78mm
Distance (D2) between two adjacent dots in Cross Direction (CD):1.1mm

The invention is however not limited to this particular bonding pattern of figures 5 to 7. In particular, the bonding dots 210a can have different shapes (round shape, square shape, rectangular shape, etc...), and one bonding pattern can be constituted by a combination of bonding dots 210a of different shapes.

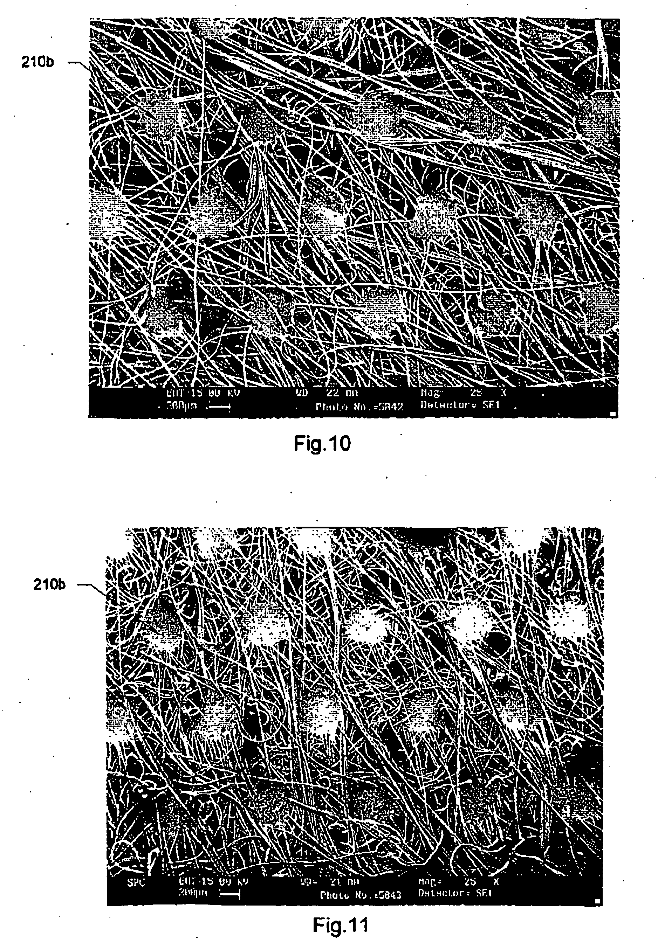

Figure 10 shows a photography of an example of a pre-consolidated spunbonded web A of the invention, having a basis weight of 14g/m$^2$. This pre-consolidated spunbonded web is made of spun filaments (F) that are made of homopolymer of polypropylene, and that have the so-called "papillon" cross section of figure 12C, and a diameter DI of 12$\mu$m. This spunbonded web was thermo-bonded with a calendar unit 2, using the aforesaid microbonding pattern C#1,

Referring to this figure 10, the pre-consolidated spunbonded layer A, issued from the thermal bonding unit 2, comprises a high number of very small bonded dots 210b, that corresponds to the bonding pattern of the engraved roll 21, and wherein the spun microfilaments are locally fused at their surface. The density (DD) of these bonded dots 210b is the same than the density of the bonding dots 210a ($\geq$ 90 bonded dots/cm$^2$). The area of each bonded dots 210b of the pre-consolidated spunbonded layer A is preferably equal or less than the area of the corresponding bonding dot 210a of the bonding pattern. The bonding ratio (R') of the pre-consolidated spunbonded layer A is preferably equal or less than the bonding ratio (R) of the bonding pattern. This bonding ratio R' is given by the following formula:

$$(4) \quad R' = \frac{\sum S_i}{S} \times 100$$

Wherein:

- S is whole area of a sample of the pre-consolidated spunbonded layer (A),
- $S_i$ is the area of each individual bonded dot of said sample

## Air-laid unit 3

The traditional air-laid unit 3, which is mounted downstream the thermal bonding unit 2, is disclosed in details, for example, in European patent application EP 0 032 772, Said air-laid unit 3 is fed with loose pulp fibers, and more preferably with short wood pulp fibers.

The pre-consolidated spunbonded web A issued from thermal bonding unit 2 is transferred continuously to a second belt 30 where pulp fibers are laid down, using a conventional air-laid process, by means of said traditional air-laid unit 3.

Preferably, the air-laid unit 3 is set up in order to produce a pulp layer B whose weight is between 15 g/m$^2$ and 50 g/m$^2$.

At the output of the air-laid unit 3, a composite (A/B) made of a pre-consolidated spunbonded web A and of an absorbent pulp top layer B is obtained.

## Carding unit 4

The carding unit 4, which is mounted between the air-laid unit 3 and the hydraulic needling unit 5, is used for producing in line a carded nonwoven cover layer C. Said carded nonwoven cover layer C issued from the carding unit 4 is laid down onto the top surface of the absorbent pulp layer B of the composite nonwoven (A/B) issued from the air-laid unit 3.

Preferably, the carding unit 4 is set up in order to produce a carded layer C, whose weight is between 10 g/m$^2$ and 30 g/m$^2$.

Hydraulic needling unit 5

The composite nonwoven (A/B/C) is transported, downstream the carding unit 3, by means of a third conveyor belt 50 through the hydraulic needling unit 5. This hydraulic needling unit 5 is used for consolidating the nonwoven composite (A/B/C), by means of high pressure water jets (hydroentanglement process) that are directed at least towards the surface of the top layer (cover layer C), and that penetrate through the structure of the composite and are partially reflected back to the structure, in order to bind the layers (A, B and C) together.

In the particular example of figure 2, the water needling process is performed on both sides of the composite nonwoven (A/B/C).

More particularly, in the example of figure 2, the hydraulic needling unit 5 comprises four successive perforated drums. First perforated drum 51 is associated with two successive hydro-jet beams 51a and 51b. Second perforated drum 52 is associated with two successive hydro-jet beams 52a and 52b. Third perforated drum 53 is associated with two successive hydro-jet beams 53a and 53b. Fourth perforated drum 54 is associated with two successive hydro-jet beams 54a and 54b. The water pressure of the upstream hydro-jet beam 51a is lower than the water pressure of all the other downstream hydro-jet beams 51b, 52a, 52b, 53a, 53b, 54a, 54b, in order to obtain a pre-hydroentanglement of the layers.

At the exit of hydraulic needling unit 5, a hydroentangled and absorbent composite A/B/C is obtained.

Dewatering unit 6

This hydroentangled and absorbent composite A/B/C is transported downstream the hydraulic needling unit 5 by the conveyor belt 60 of a dewatering unit 6, and over a vacuum box 61, that enables to remove by suction from the composite A/B/C most of the water that has been absorbed during the water needling process (conventional dewatering process).

The hydroentanglement unit 5 and the dewatering unit 6 can be integrated in the same industrial equipment.

Drying unit 7

The dewatered hydroentangled absorbent composite nonwoven A/B/C issued from the dewatering unit 6 is continuously fed through the oven of the drying unit 7, wherein heat is applied to the composite (for example by means of hot air), in order to remove the remaining water still contained within the composite nonwoven.

Winding unit 8

Then the dried composite nonwoven A/B/C is wound in the form of a roll, by means of the winding unit 8.

Preferably, but not necessarily, the weight of said hydroentangled and absorbent composite A/B/C is between 27 g/m$^2$ and 115 g/m$^2$.

**EXAMPLES N°1 to N°22**

The invention will now be illustrated by the following non-limiting examples.

**Examples N°1 to N°14/ Pre-consolidated spunbonded web**

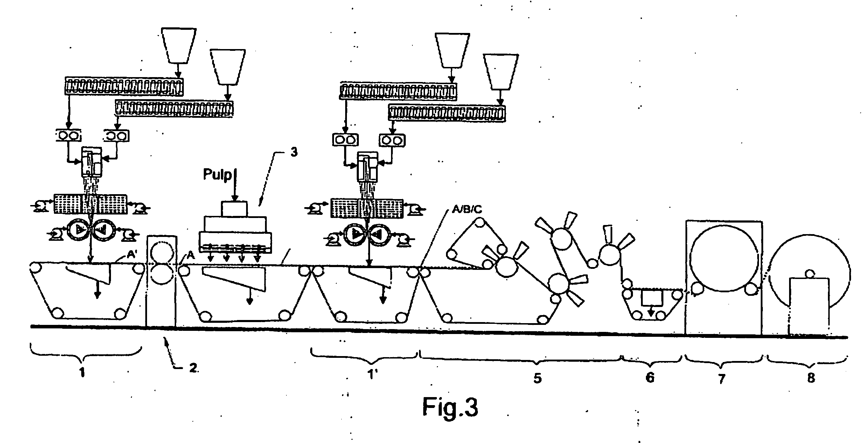

Several samples (Examples N° 1 to N°14) of a pre-consolidated spunbonded web A were produced with the spunbonding unit 1 and thermal bonding unit 2 of figures 2 or 3. The main production data for each sample N°1 to 14 are summarized hereafter in tables 1A, 1B and 1C.

Table 1A : Spunbonded production data - Spinning

| | RAW MATERIALS | | FILAMENT SPINNING [Filaments F'- Spunbonding unit (1)] | | | | |
|---|---|---|---|---|---|---|---|
| Ex | Polymer(s) type(s) | Polymer(s) percentage (s) | Spinneret type (holes per meter / hole diameter) | filaments section shape | filaments diameter DI | filaments count CT (dtex) | Through put (ghm) |
| N° | --- | [%] | [holes per m / mm] | --- | [μm] | [g/10000m] | [g/hole/ min] |
| 1 | PP[1] | 100 | 5000/0,35 | round | 9,5 | 0,65 | 0,33 |
| 2 | PP[1] | 100 | 5000/0,35 | round | 12,4 | 1,10 | 0,33 |
| 3 | PP[1] | 100 | 5000/0,35 | round | 13,5 | 1,30 | 0,33 |
| 4 | PP[1] | 100 | 5000/0,35 | round | 17,1 | 2,10 | 0,33 |
| **5** | **PP[2]** | **100** | **6000/0,35** | **round** | **9,7** | **0,67** | **0,29** |
| **6** | **PP[2]** | **100** | **6000/0,35** | **round** | **12,3** | **1,08** | **0,29** |
| **7** | **PP[2]** | **100** | **6000/0,35** | **round** | **13,3** | **1,27** | **0,29** |
| 8 | PP[2] | 100 | 6000/0,35 | round | 16,9 | 2,05 | 0,29 |
| 9 | PP[1] | 100 | 5000/0,35 | round | 9,7 | 0,67 | 0,35 |
| 10 | PP[1] | 100 | 5000/0,35 | round | 13,8 | 1,37 | 0,35 |
| 11 | PP[1] | 100 | 5000/0,35 | round | 16,7 | 2,00 | 0,35 |
| **12** | **PP[2]** | **100** | **6000/0,35** | **round** | **9,6** | **0,66** | **0,29** |
| **13** | **PP[2]** | **100** | **6000 / 0,35** | **round** | **13,6** | **1,33** | **0,29** |
| 14 | PP[2] | 100 | 6000 / 0,35 | round | 17,1 | 2,10 | 0,29 |

PP[1]: Polypropylene Borealis HH450FB
PP[2]: Polypropylene TOTAL PPH10099

Table 1B : Spunbonded production data - Web Forming

| | WEB FORMING [Filaments F-Spunbonding unit (1)] | | |
|---|---|---|---|
| Ex | Draw slot Pressure | Line Speed | Nonwoven Basis Weigth |
| N° | [bar] | [m/min] | [g/m²] |
| 1 | 2,1 | 167 | 10 |
| 2 | 1,5 | 167 | 10 |
| 3 | 1,2 | 167 | 10 |
| 4 | 0,8 | 167 | 10 |
| **5** | **2** | **175** | **10** |
| **6** | **1,4** | **175** | **10** |
| **7** | **1,2** | **175** | **10** |
| 8 | 0,7 | 175 | 10 |
| 9 | 2,1 | 55 | 32 |
| 10 | 1,2 | 55 | 32 |
| 11 | 0,8 | 55 | 32 |

(continued)

| | WEB FORMING [Filaments F-Spunbonding unit (1)] | | |
|---|---|---|---|
| Ex | Draw slot Pressure | Line Speed | Nonwoven Basis Weigth |
| N° | [bar] | [m/min] | [g/m²] |
| 12 | 2 | 55 | 32 |
| 13 | 1,2 | 55 | 32 |
| 14 | 0,7 | 55 | 32 |

Table 1C : Spunbonded production data - Web Bonding

| | WEB BONDING [Thermal bonding unit (2)] | | | | | |
|---|---|---|---|---|---|---|
| Ex | Calender Type | Calender Bonding Ratio (R) | Calender Dots density | Engraved Roll (21) Temperature | Smooth Roll (20) Temperature | Calender Linear Pressure |
| N° | __ | [%] | [dots/cm²] | [°C] | [°C] | [N/mm] |
| 1 | C#2 | 18 | 32,8 | 120 | 120 | 40 |
| 2 | C#2 | 18 | 32,8 | 120 | 120 | 40 |
| 3 | C#2 | 18 | 32,8 | 120 | 120 | 40 |
| 4 | C#2 | 18 | 32,8 | 120 | 120 | 40 |
| 5 | C#1 | 18,5 | 102 | 120 | 120 | 40 |
| 6 | C#1 | 18,5 | 102 | 120 | 120 | 40 |
| 7 | C#1 | 18,5 | 102 | 120 | 120 | 40 |
| 8 | C#1 | 18,5 | 102 | 120 | 120 | 40 |
| 9 | C#2 | 18 | 32,8 | 140 | 140 | 60 |
| 10 | C#2 | 18 | 32,8 | 140 | 140 | 60 |
| 11 | C#2 | 18 | 32,8 | 140 | 140 | 60 |
| 12 | C#1 | 18,5 | 102 | 140 | 140 | 60 |
| 13 | C#1 | 18,5 | 102 | 140 | 140 | 60 |
| 14 | C#1 | 18,5 | 102 | 140 | 140 | 60 |

In all examples N°1 to N°14, the filaments of the spunbonded web were round monocomponent filaments made of homopolymer of polypropylene. In Examples N°1 to N°8, the weight of the spunbonded web was 10 g/m² ; In Examples N°9 to N°14, the weight of the spunbonded web was 32 g/m².

Examples N°5 to N°7 and Examples N° 12 and N°13/Invention

Examples N°5 to N°7 (10 g/m²) and examples N° 12 and N°13 (32 g/m²) relate to pre-consolidated spunbonded webs of the invention, which have been compressed and thermo-bonded with the same engraved roll 21 having the previously described micro-bonding pattern "C#1". They differ from each other by the diameter (DI) of their continuous spun microfilaments (F).

Comparative Examples N°1 to 4 and N°8 to N°11, and N°14

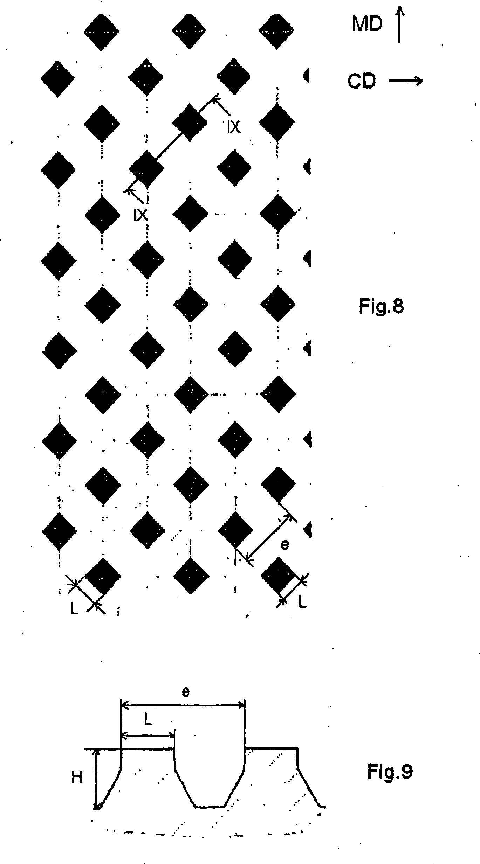

Examples N°1 to N°4 (10 g/m²) and examples N°9 to N°11 (32 g/m²) relate to spunbonded webs which have been compressed and thermo-bonded with the same engraved roll 21, said engraved roll 21 having the bonding pattern of figures 8 and 9. The main dimensions of this bonding pattern (referred therein "C#2") were the followings:

Shape of the bonding dots: square
Density of bonding dots: 32.8 dots/cm$^2$
Bonding ratio (R):18%
Dot width (L): 0.74mm
Dot area (DA): 0.5476 mm$^2$
Dot height (H): 0.8 mm
Distance (e) between two adjacent dots:1.747mm

In respect of the low density of bonding dots (32.8 dots/cm$^2$) and of the large area of each bonding dots (0.5476mm$^2$), said bonding pattern "C#2" is outside the scope of the invention, and these examples N°1 to N°4 (10 g/m$^2$) and examples N°9 to N°11 (32 g/m$^2$) are thus comparative examples, not covered by the invention.

Example N°8 (10 g/m$^2$) and example N°14 (32 g/m$^2$) relate to pre-consolidated spunbonded webs, which have been thermo-bonded with microbonding pattern "C#1", but which are made of continuous spun filaments (F), having a diameter (DI) higher than 15$\mu$m (i.e. outside the scope of the invention).

Air permeability test

For each example N°1 to N°14, the air permeability of the pre-consolidated spunbonded web was measured according to the following method.

Air Permeability Test was performed on a Textest model FX 3300 available from the Textest Instruments - Zurich, according to WSP 70.1 (05) standard. The rate of air flow passing perpendicularly through a given area of fabric is measured at a given pressure difference across the fabric test area over a given time period. The specimen (a single layer) was positioned in the circular specimen holder, with an orifice allowing the test to be carried out on an area of 38cm$^2$. The pressure gap was set at 125 Pa.

The air permeability results (expressed in m$^3$/m$^2$/min) for examples N°1 to N°14 are summarized hereafter in tables 2A, 2B, 3A and 3B.

Table 2A : Spunbonded web -10 g/m$^2$- Air permeability versus spun filament diameter

| | | | | NONWOVEN | | |
| --- | --- | --- | --- | --- | --- | --- |
| | Count CT (dtex) | Diameter DI | Filaments Lateral Area (1 filament length=10000m) | Basis weight | Equivalent Area (Filaments Lateral Area in 1m$^2$ of Nonwoven) | Air Permeability Ref. Method: WSP 70.1 (05) |
| Ex | A | B | C = (B / 10$^6$) $\times$ 10000 | D | E=(C / A) $\times$ D | - |
| N° | [g/10000m] | [$\mu$m] | [m$^2$] | g/m$^2$ | [m$^2$/m$^2$] | [m$^3$/(m$^2$ x min)] |
| 1 | 0,65 | 9,5 | 0,10 | 10 | 1,47 | 253 |
| 2 | 1,10 | 12,4 | 0,12 | 10 | 1,13 | 266 |
| 3 | 1,30 | 13,5 | 0,13 | 10 | 1,04 | 279 |
| 4 | 2,10 | 17.1 | 0,17 | 10 | 0,82 | 370 |

Table 2B : Spunbonded web -32 g/m$^2$- Air permeability versus spun filament diameter

| | FIBER | | | NONWOVEN | | Air Permeability Ref. Method: WSP 70.1 (05) |
|---|---|---|---|---|---|---|
| | count CT(dtex) | Diameter | Filaments Lateral Area (1 filament. length=10000m) | Basis weight | Equivalent Area (Filaments Lateral Area In 1m$^2$ of Nonwoven) | |
| Ex | A | B | C = (B/10$^6$) $\times$10000 | D | E=(C/A) $\times$ D | - |
| N° | [g/10000m] | [μm] | [m$^2$] | g/m$^2$ | [m$^2$/m$^2$] | [m$^3$/(m$^2$ x min)] |
| 9 | 0.67 | 9,7 | 0,10 | 32 | 4.63 | 77 |
| 10 | 1,37 | 13,8 | 0,14 | 32 | 3.23 | 88 |
| 11 | 2,00 | 16,7 | 0,17 | 32 | 2,68 | 101 |

Table 3A : Spunbonded web -10 g/m$^2$- Air permeability versus calender design (population dots)

| | FILAMENT | NONWOVEN | CALENDER (DESIGN) | | | Air Permeability Ref. Method: WSP 70.1 (05) |
|---|---|---|---|---|---|---|
| | Diameter (DI) | Basis weight | Type | Bonding Ratio (R) | Dots density (DD) | |
| Ex | [μm] | [g/m$^2$] | -- | [%] | [dots/cm$^2$] | [m$^3$/(m$^2$ x min)] |
| 1 | 9,5 | 10 | C#2 | 18.0 | 32,8 | 253 |
| 5 | 9,7 | 10 | C#1 | 18,5 | 102 | 224 |
| 2 | 12,4 | 10 | C#2 | 16,0 | 32,8 | 266 |
| 6 | 12,3 | 10 | C#1 | 18,5 | 102 | 240 |
| 3 | 13,5 | 10 | C#2 | 18,0 | 32,8 | 279 |
| 7 | 13,3 | 10 | C#1 | 18,5 | 102 | 261 |
| 4 | 17,1 | 10 | C#2 | 18,0 | 32,8 | 370 |
| 8 | 16,9 | 10 | C#1 | 18,5 | 102 | 329 |

Table 3B : Spunbonded web - 32g/m$^2$- Air permeability versus calender design (population dots)

| | FIBRE | NONWOVEN | CALENDER (DESIGN) | | | Air Permeability Ref. Method: WSP 70.1 (05) |
|---|---|---|---|---|---|---|
| EX | diameter (DI) | Basis weight | Type | Bonding Ratio (R) | Dots density (DD) | |
| N° | [μm] | [g/m$^2$] | - | [%] | [dots/cm$^2$] | [m$^3$/(m$^2$ x min)] |
| 9 | 9,7 | 32 | C#2 | 18 | 32,8 | 77 |
| 12 | 9,6 | 32 | C#1 | 18,5 | 102 | 68 |
| 10 | 13.8 | 32 | C#2 | 18 | 32,8 | 88 |
| 13 | 13.6 | 32 | C#1 | 18,5 | 102 | 80 |
| 11 | 16,7 | 32 | C#2 | 18 | 32.8 | 101 |
| 14 | 17,1 | 32 | C#1 | 18.5 | 102 | 94 |

The air permeability measurements show that the air permeability of the pre-consolidated spunbonded web A increases with the spun filament diameter (DI) and decreases with the density (DD) of the bonding dots. Pre-consolidated spunbonded webs of the invention (Examples N°5, N°6, N°7, N°12 and N°13) advantageously exhibit lower air permeability, and the spunbonded layer constitutes therefore an improved barrier for the pulp fibres during the water needling process of the composite nonwoven.

**Examples N°15 to N°22/ Composite nonwoven A/B/C**

Several samples (Examples N° 15 to N°22) of a three-layered hydroentangled and absorbent composite nonwoven (A/B/C) were produced by means of a continuous system like the one previously described and shown on figure 2. The main production data for these examples N°15 to 22 are summarized hereafter in tables 4A, 4B and 4C.

The basis weight of the composite nonwoven of examples N°15 to 18 was 45 g/m$^2$. The basis weight of the composite nonwoven of examples N°19 to 22 was 90 g/m$^2$.

Examples N°15, N°16, N°19 and N°20 relate to hydroentangled and absorbent composite nonwoven comprising a spunbonded layer A that is the same respectively than examples N°2, N°1, N°10 and N°9, and are thus comparative examples not covered by the invention.

Examples N°17, N°18, N°21 and N°22 relate to hydroentangled and absorbent composite nonwoven comprising a spunbonded layer A that is the same respectively than examples N°5, N°6, N°13 and N°12, and are thus covered by the invention.

Table 4A : Composite nonwoven A/B/C (Spun/Pulp/Carded)-Production data

| | SPC Basis Weight (A/B/C) | | | | SPUN BONDED (A) | PULP (B) | CARDED (C) |
|---|---|---|---|---|---|---|---|
| | | | | | RAW MATERIALS | | |
| Ex | Total | A | B | C | Spunbonded type | Pulp type | Fibre(s) type(s) |
| N° | [g/m$^2$] | [g/m$^2$] | [g/m$^2$] | [g/m$^2$] | Ex | - | - |
| 15 | 45 | 10 | 22 | 13 | N°2 | (I) | (II) |
| 16 | 45 | 10 | 22 | 13 | N°1 | (I) | (II) |
| **17** | **45** | **10** | **22** | **13** | **N°5** | **(I)** | **(II)** |
| **18** | **45** | **10** | **22** | **13** | **N°6** | **(I)** | **(II)** |
| 19 | 90 | 32 | 33 | 25 | N°10 | (I) | (III) |
| 20 | 90 | 32 | 39 | 25 | N°9 | (I) | (III) |
| **21** | **90** | **32** | **33** | **25** | **N°13** | **(I)** | **(III)** |
| **22** | **90** | **32** | **33** | **25** | **N12** | **(I)** | **(III)** |
| (I) Weyerhauser, NF 405  (II) PP Arborea Perm. Phil., 1,7 dtex, 38 mm  (III) PP Arborea Perm. Phil., 1,7 dtex, 38mm (50%wt)/ Lyocell, Lenzing Tencel, 1,7dtex, 38mm (50%wt) | | | | | | | |

Table 4B : Composite nonwoven A/B/C (Spun/Pulp/Carded)-Hydroentanglement step

| Ex. N° | HYDROENTAGLEMENT (UNIT 5) | | | | | | | | |
|---|---|---|---|---|---|---|---|---|---|
| | Beam (51a) | Beam (51b) | Beam (52a) | Beam (52b) | Beam (53a) | Beam (53b) | Beam (54a) | Beam (54b) | Pattern type (54) |
| | [bar] | [bar] | [bar] | [bar] | [bar] | [bar] | [bar] | [bar] | _ |
| 15 | 15 | 30 | 70 | 70 | 45 | 45 | _ | _ | None |
| 16 | 15 | 30 | 65 | 65 | 50 | 50 | _ | _ | None |
| 17 | 15 | 35 | 90 | 90 | 70 | _ | 70 | _ | None |
| 18 | 15 | 35 | 90 | 90 | 70 | _ | 70 | _ | None |
| 19 | 15 | 50 | 130 | 130 | 70 | 70 | _ | _ | none |
| 20 | 15 | 50 | 130 | 130 | 70 | 70 | _ | _ | none |

(continued)

| Ex. N° | HYDROENTAGLEMENT (UNIT 5) | | | | | | | | |
|---|---|---|---|---|---|---|---|---|---|
| | Beam (51a) | Beam (51b) | Beam (52a) | Beam (52b) | Beam (53a) | Beam (53b) | Beam (54a) | Beam (54b) | Pattern type (54) |
| | [bar] | [bar] | [bar] | [bar] | [bar] | [bar] | [bar] | [bar] | _ |
| 21 | 20 | 80 | 70 | 70 | 100 | 100 | _ | _ | none |
| 22 | 20 | 80 | 70 | 70 | 100 | 100 | _ | _ | none |

Table 4C : Composite nonwoven A/B/C (Spun/Pulp/Carded)- Drying and winding step

| | DRYING (Unit 7) | WINDING (Unit 8) |
|---|---|---|
| Ex. N° | Oven Temperature | line speed |
| | [°C] | [m/min] |
| 15 | 110 | 40 |
| 16 | 110 | 40 |
| 17 | 165 | 187 |
| 18 | 165 | 187 |
| 19 | 110 | 30 |
| 20 | 110 | 30 |
| 21 | 130 | 59 |
| 22 | 130 | 59 |

Different tests have been performed on samples of examples N°15 to 22.

**Abrasion resistance Test**

Abrasion resistance testing was performed on a Martindale Abrasion Tester (Model: Nu-Martindaie Abrasion and Pilling tester from James H. Heal & Co. Ltd - Halifax, England). Tests were performed according to ASTM-D-4966-98 using a pressure of 12 kilopascals (KPa) on the spunbonded side (A) of the composite AIB/C.
Samples were subjected to 150 cycles and were then examined for the presence of surface fuzzing, pilling, roping or holes. The samples were compared to a visual scale and assigned a wear number from 1 to 5, wherein wear number 5 was indicating little or no visible abrasion and wear number 1 was indicating a hole worn through the sample.
The results of the abrasion resistance test are summarized hereafter in table 5A (basis weight 45 g/m$^2$) and in table 5B (basis weight 90 g/m$^2$).
The pre-consolidated spunbonded layers A of examples N°17, 18, 21 and 22 (invention) have advantageously a better abrasion resistance than the pre-consolidated spunbonded layers of the other comparative examples. The comparison between examples having the same basis weight and having spun filaments of similar diameter (Ex. N°15 vs. Ex. N°18; EX N°16 vs. Ex. N°17 ; EX. N°19 vs. Ex. N°21 ; EX. N°20 vs. Ex. N°22) further shows that the microbonding pattern of the invention, with very high density of bonding dots (DD), improves the abrasion resistance of the spunbonded layer A, compared with the use of a bonding pattern having a low density of bonding dots (DD).

Table 5A : Composite SPC - 45 g/m$^2$- Abrasion resistance

| SPC (A/B/C) | | SPUNBONDED (A) | | | MARTINDALE ABRASION Ref. Method: ASTM D4966-98 |
|---|---|---|---|---|---|
| Ex. | Basis weight | A | Filament diameter (DI) | Calender Dots density (DD) | Fabric side tested: spunbonded |
| N° | [g/m$^2$] | Ex. | [µm] | [dots/cm$^2$] | [values; from 1 to 5] |
| 15 | 45 | N°2 | 12.4 | 32,8 | 3,25 |
| 16 | 45 | N°1 | 9.5 | 32,8 | 3.50 |
| 18 | 45 | N°6 | 12,3 | 102 | 4,00 |
| 17 | 45 | N°5 | 9,7 | 102 | 4,25 |

Table 5B: Composite SPC - 90 g/m$^2$- Abrasion resistance

| SPC (A/B/C) | | SPUNBONDED | | | MARTINDALE ABRASION Ref. Method; ASTM D4966-98 |
|---|---|---|---|---|---|
| Ex | Basis weight | A | Filament diameter (DI) | Calender Dots density (DD) | Fabric side tested, spunbonded |
| N° | [g/m$^2$] | Ex. | [µm] | [dots/cm$^2$] | [values: from 1 to 5] |
| 19 | 90 | N°10 | 13,8 | 32,8 | 4.00 |
| 20 | 90 | N°9 | 9,7 | 32,8 | 4,25 |
| **21** | **90** | **N°13** | **13,6** | **102** | **4,50** |
| **22** | **90** | **N°12** | **9,6** | **102** | **4,75** |

**Handle-O-Meter Test**

Handle-O-Meter testing was performed on a Handle-O-Meter Model no. 211-5, model 211-2001 available from the Thwing-Albert Company. Tests were performed according to WSP 90.3.0 (05) standard. The nonwoven to be tested is deformed through a restricted opening by a plunger and the required force corresponds to the surface friction of the nonwoven.

The determination of the combined effects of stiffness and thickness is correlated with finished product properties like softness. A slot width of 6.4mm and square specimen (200mm x 200mm) were used. Three specimens for each sample were tested. Each specimen was tested on both sides, spunbonded (side1) and carded (side2) and in both directions, Machine directions (MD) and Cross direction (CD). Therefore, the total number of measurements for each specimen was 4. Results include average value MD and CD (side1, side 2) and the "Total Hand" (sum of: MD side1. MDside2, CD side1, CD side2).

The results of the handle-o-meter test are summarized hereafter in table 6A (basis weight 45 g/m$^2$) and in table 6B (basis weight 90 g/m$^2$). Table 6A : Composite SPC - 45 g/m$^2$- stiffness (handle-o-meter test)

| SPC (A/B/C) | HANDLE-O-METER Ref. Method: WSP 90.3.0 (05) | | | | | | |
|---|---|---|---|---|---|---|---|
| Ex | Specimen | Side 1 up. MD | Side 1 up. CD | Side 2 up. MD | Side 2 up. CD | Average, MD | Average, CD | Total "Hand" |
| | | A1 | B1 | C1 | D1 | (A1+C1)/2 | (B1+D1)/2 | A1+B1+C1+D1 |
| N° | N | [cN] | [cN] | [cN] | [cN] | [cN] | [cN] | [cN] |

(continued)

| SPC (A/B/C) | HANDLE-O-METER Ref. Method: WSP 90.3.0 (05) | | | | | | | |
|---|---|---|---|---|---|---|---|---|
| Ex | Specimen | Side 1 up. MD | Side 1 up. CD | Side 2 up. MD | Side 2 up. CD | Average, MD | Average, CD | Total "Hand" |
| 15 | 1 | 60.8 | 15.2 | 70.2 | 12.6 | 65.5 | 13.9 | 168.8 |
| | 2 | 67.1 | 11.8 | 62.7 | 11.9 | 64.9 | 11.9 | 153.5 |
| | 3 | 74.4 | 12.8 | 69.1 | 12.1 | 71.8 | 12.5 | 168.4 |
| | average | | | | | 87.4 | 12.7 | 160.2 |
| 16 | 1 | 51.9 | 12.1 | 54.1 | 10.1 | 53.0 | 11.1 | 128.2 |
| | 2 | 58.0 | 11.5 | 52.0 | 8.6 | 55.0 | 10.1 | 130.1 |
| | 3 | 57.8 | 11.9 | 52.1 | 9.0 | 85.0 | 10.5 | 130.8 |
| | average | | | | | 54.3 | 10.5 | 129.7 |
| 18 | 1 | 49.0 | 10.3 | 49.3 | 7.7 | 49.2 | 9.0 | 116.3 |
| | 2 | 52.1 | 10.7 | 51.1 | 7.3 | 51.6 | 9,0 | 121.2 |
| | 3 | 50.0 | 11.0 | 50,4 | 9,0 | 50,2 | 10.0 | 120.4 |
| | average | | | | | 50.3 | 9.3 | 119.3 |
| 17 | 1 | 44.5 | 9.9 | 49,2 | 6.8 | 46.9 | 8.4 | 110,4 |
| | 2 | 43.0 | 9.0 | 46.0 | 6.7 | 46.6 | 7.9 | 106.7 |
| | 3 | 47.1 | 9.3 | 46.2 | 7.0 | 46.7 | 8.2 | 109.8 |
| | average | | | | | 46.3 | 8.1 | 108.8 |

Table 6B : Composite SPC - 90 g/m$^2$- stiffness (handle-o-meter test)

| SPC (A/B/C) | HANDLE-O-METER Ref. Method: WSP 90,3.0 (05) | | | | | | | |
|---|---|---|---|---|---|---|---|---|
| EX | Specimen | Side 1 up, MD | Side 1 up, CD | Side 2 up, MD | Side 2 up, CD | Average, MD | Average, CD | Total "Hand" |
| | | A1 | B1 | C1 | D1 | (A1+C1)/2 | (B1+D1)/2 | A1+B1+C1+D1 |
| N° | N | [cN] | [cN] | [cN] | [cN] | [cN] | [cN] | [cN] |
| 19 | 1 | 91.9 | 19,8 | 94,0 | 21,1 | 93,0 | 20.5 | 226,8 |
| | 2 | 90.1 | 18,4 | 93.1 | 23,3 | 91,6 | 20,9 | 224,9 |
| | 3 | 90,0 | 18,7 | 93.3 | 22.0 | 91,7 | 20.4 | 224,0 |
| | **average** | | | | | **92,1** | **20.6** | **225,2** |
| 20 | 1 | 74,0 | 16,1 | 80,0 | 17,0 | 91,8 | 20.6 | 187,1 |
| | 2 | 70,3 | 15,8 | 81,1 | 16.4 | 75,7 | 16,1 | 183,6 |
| | 3 | 72,4 | 15,9 | 78,5 | 17,9 | 75,5 | 16,9 | 184,7 |
| | **average** | | | | | **81,0** | **17,9** | **185,1** |

(continued)

| SPC (A/B/C) | HANDLE-O-METER Ref. Method: WSP 90,3.0 (05) | | | | | | |
|---|---|---|---|---|---|---|---|
| EX | Specimen | Side 1 up, MD | Side 1 up, CD | Side 2 up, MD | Side 2 up, CD | Average, MD | Average, CD | Total "Hand" |
| | | A1 | B1 | C1 | D1 | (A1+C1)/2 | (B1+D1)/2 | A1+B1+C1+D1 |
| 21 | 1 | 31,7 | 12,8 | 53,3 | 15,7 | 42,5 | 14,3 | 113,5 |
| | 2 | 32,9 | 13,1 | 49,3 | 17,1 | 41,1 | 15,1 | 112,4 |
| | 3 | 32,9 | 13,1 | 49,3 | 17,1 | 41,1 | 15,1 | 112,4 |
| | average | | | | | **41,6** | **14,8** | **112,8** |
| 22 | 1 | 29,0 | 11,4 | 47,3 | 14,0 | 41,3 | 15,0 | 101,7 |
| | 2 | 28,4 | 12,0 | 49,3 | 14,5 | 41,3 | 15,0 | 104,2 |
| | 3 | 27,3 | 13,2 | 47,1 | 13,4 | 41,4 | 14,9 | 101,0 |
| | average | | | | | **41,3** | **15,0** | **102,3** |

The composite nonwovens (A/B/C) of examples N°17, 18, 21 and 22 (invention) have advantageously a lower stiffness both in CD and MD directions (and thus a higher softness) than the other comparative examples. The comparison between examples having the same basis weight and having spun filaments of similar diameter (Ex. N°15 vs. Ex. N°18; EX. N°16 vs. Ex. N°17 ; EX. N°19 vs. Ex. N°21 ; EX. N°20 vs. Ex. N°22) shows that the microbonding pattern of the invention, with very high density of bonding dots (DD), improves the softness of the final product, compared with the use of a bonding pattern having a low density of bonding dots (DD).

**Tensile properties test**

Tensile tests were performed on a dynamometer model 5564 available from Instron Instruments, according to WSP 110.4 (05) standard.

Tensile strength refers to the maximum load (i.e., peak load) encountered while elongating the sample to break. Elongation at peak is the specimen elongation that corresponds to the peak load. Measurements were made in cross-direction on dry samples.

Specimens were cut 25mm width and 125mm length. The distance between the clamps of the dynamometer was set at 75mm and the traction speed was set at 300mm/min.

The results of the tensile properties test are summarized hereafter in table 7A (basis weight 45 g/m$^2$) and in table 7B (basis weight 90 g/m$^2$).

Table 7A : Composite SPC-45 g/m$^2$- Tensile properties (MD, CD)

| SPC(A/B/C) | | SPUNBONDED | | | TENSILE PROPERTIES Ref. Method: WSP 110.4 (05) | | | |
|---|---|---|---|---|---|---|---|---|
| Ex | Basis weight | A | Filament diameter (DI) | Calender Dots density (DD) | Load at Peak. MD | Load at Peak, CD | Elongation at peak, MD | Elongation at Peak, CD |
| N° | [g/m$^2$] | EX | [μm] | [dots/cm$^2$] | [N] | [N] | [%] | [%] |
| 15 | 45 | N°2 | 12,4 | 32,8 | 49,0 | 5,6 | 57 | 73 |
| 16 | 46 | N°1 | 9,5 | 32,8 | 53,3 | 8.9 | 60 | 123 |
| 18 | 45 | N°8 | 12,3 | 102 | 50.1 | 9,0, | 62 | 110 |
| 17 | 45 | N°5 | 9.7 | 102 | 57,0 | 9,5 | 59 | 127 |

Table 7B: Composite SPC - 45 g/m$^2$- Tensile properties (MD, CD)

| SPC (A/B/C) | | SPUNBONDED | | | TENSILE PROPERTIES Ref. Method: WSP 110.4 (05) | | | |
|---|---|---|---|---|---|---|---|---|
| EX | Basis weight | A | Filament diameter (DI) | Calender Dots density (DD) | Load at Peak, MD | Load at Peak, CD | Elongation at Peak, MD | Elongation at Peak, CD |
| N° | [g/m$^2$] | EX | [μm] | [dots/cm$^2$] | [N] | [N] | [%] | [%] |
| 19 | 90 | N°10 | 13,8 | 32,8 | 82,7 | 19,3 | 35 | 54 |
| 20 | 90 | N°9 | 9,7 | 32,8 | 87,1 | 31,1 | 62 | 99 |
| 21 | 90 | N°13 | 13,6 | 102 | 92,2 | 39,1 | 79 | 104 |
| 22 | 90 | N°12 | 9,6 | 102 | 96,3 | 43,1 | 82 | 111 |

The comparison between examples having the same basis weight and having spun filaments of similar diameter (Ex. N°15 vs. Ex. N°18; EX. N°16 vs. Ex. N°17 ; EX. N°19 vs. Ex. N°21 ; EX. N°20 vs. Ex. N°22) shows that the microbonding pattern of the invention, with very high density of bonding dots (DD), improves these tensile properties of the final product, especially load at peak both in CD and MD directions, compared with the use of a bonding pattern having a low density of bonding dots (DD).

In addition to the above properties, it has been further noticed that during the water needling process of the composite nonwovens (A/B/C) of examples N°17, 18, 21 and 22 (invention), the pulp fibres forming the absorbent layers were only slightly washed through the pre-consolidated spunbonded layer A, and were thus retained for the useful effect of the product. This reduction of pulp loss can be explained by the very small pore size (or otherwise stated the lower air permeability) of the said pre-consolidated spunbonded layer (A) of the invention.

By way only of non-limiting example, figure 11 shaws a photography of a sample of a hydroentangled absorbent composite nonwoven (A/B/C) of the invention, having a basis weight of 50 g/m$^2$, and wherein:

-    layer A is a pre-consolidated spunbonded web made of round shape spun filament (F) having a diameter of 10.5μm, and made of homopolymer of polypropylene,
-    layer B is a pulp layer
-    layer C is a carded layer,

The spunbonded layer A was thermo-bonded with a calendar unit 2, using the aforesaid microbonding pattern C#1. This photography was taken on the spunbonded side (A) of the composite nonwoven. This composite nonwoven exhibits a high uniformity.

The invention is not limited to hydroentangled composite made of three layers (A/B/C), but the absorbent composite nonwoven could be made solely of the two layers A and B.

Furthermore, in case of a three layer composite nonwoven (A/B/C), the cover layer C is not necessarily a carded layer, but can be any other nonwoven layer, and in particular a spunbonded layer. For example, in the continuous system of figure 3, the carding unit 4 of figure 2 has been replaced by a second spunbonding unit 1'. In that case, the cover layer C of the composite nonwoven is not a carded layer, but is replaced by a spunbonded layer made of continuous filaments. The spunbonded layer C can be made of spun microfilaments having a diameter DI less or equal than 15μm, or can be made of thicker spun filaments.

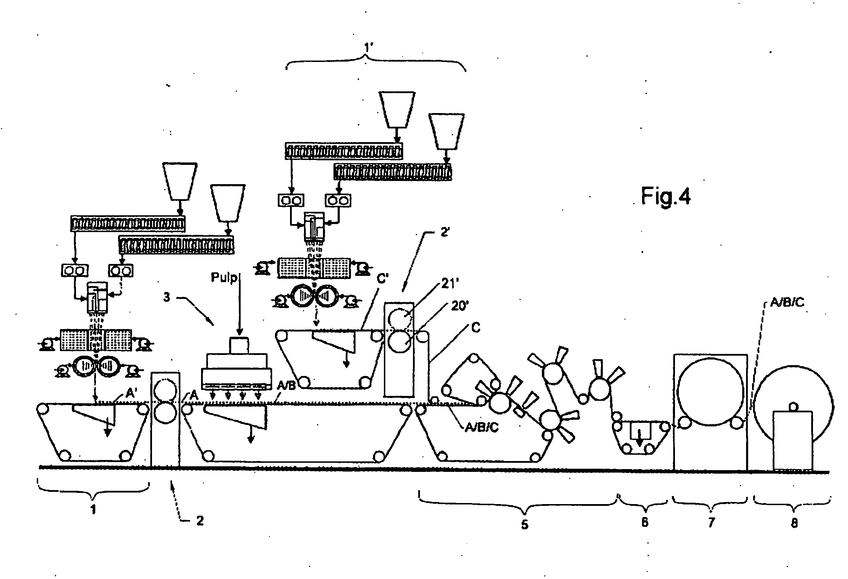

Figure 4 shows another continuous system of the invention for producing a hydroentangled and absorbent composite nonwoven made of three layers. Compared with continuous system of figure 2, in the continuous system of figure 4, the carding unit 4 of figure 2 has been replaced by a second spunbonding unit 1' and by a thermal bonding unit 2' that are similar to the previously described spunbonding unit 1 and thermal bonding unit 2. In particular, the thermal bonding unit 2' comprises two heated rolls 20' and 21'. The lower roll 20' has a smooth surface, and is for example a smooth steel roll. The upper roll 21' has an engraved surface with protruding ribs, that are regularly distributed over the whole surface of the roll, and that form a bonding pattern having bonding dots 210a (like roll 21 of thermal bonding unit 2).

The continuous system of figure 4 is used for producing a hydroentangled and absorbent composite nonwoven made of three layers: a carrier layer constituted by the pre-consolidated spunbonded layer (A) previously described for the embodiment of figure 2; an intermediate pulp layer (B) previously described for the embodiment of figure 2 ; a cover layer (C) constituted by a pre-consolidated spunbonded layer.

In a preferred embodiment, the said second spunbonding unit 1' is set up in order to produce a spunbonded web C' made of spun filaments that comprise or are constituted by very fine spun microfilaments having a diameter DI

less than or equal to 15$\mu$m, and more preferably less than or equal to 10$\mu$m. More preferably, the spunbonded web C' is a web whose weight is between 7 g/m$^2$ and 35g/m$^2$, preferably less than 25 g/m$^2$ , more preferably less than 12 g/m$^2$. The density DD of the bonding dots 21 0a of the engraved roll 21' is also very high, and at least equal to 90 bonding dots/cm$^2$, and more preferably at least equal to 100 bonding dots/cm$^2$ ; the bonding ratio (R) is low, and preferably less than 30% and more preferably less than 20%. The area DA of each bonding dot 210a of engraved roll 21' is less than 0.5mm$^2$, preferably less than 0.3mm$^2$, and more preferably less than 0.2mm$^2$. Different shapes for bonding dots 210a of the engraved roll 21' are suitable for practicing the invention (round shape, oval shape, square shape, rectangular shape ...). In this preferred embodiment, the microbonding pattern of the engraved roll 21' can be the same as the microbonding pattern of the engraved roll 21 of thermal bonding unit 2, but this is not mandatory.

The composite nonwoven (A/B/C) produced with the aforesaid preferred embodiment of continuous system of figure 4 has advantageously comparable properties on both sides of the composite nonwoven.

The composite of the invention can comprise more than three superposed layers. For example, an additional layer (for example a carded layer) could be added underneath the spunbonded layer A' ; in that case, this additional layer is for example laid onto conveyor belt 16, upstream the spunbonding unit 1, and the continuous spun filaments F are laid directly onto this additional layer.

### Claims

1.  Method of producing a hydroentangled absorbent composite nonwoven, said method comprising the steps of :

    (a) forming one spunbonded layer (A'),
    (b) thermo-bonding the said spunbonded layer (A'), in order to obtain a pre-consolidated spunbonded layer (A),
    (c) laying one absorbent pulp layer (B) onto and in contact with the pre-consolidated spunbonded layer (A),
    (d) consolidating the composite nonwoven by hydrodynamic needling,

    **characterized in that** the spunbonded layer (A') formed in step (a) comprises continuous microfilaments having a diameter (DI) less than or equal to 15 $\mu$m, and **in that** the pre-consolidation step (b) of the spunbonded layer (A') is performed by means of a bonding pattern having bonding dots (210a), the density (DD) of said bonding dots (210a) being higher than or equal to 90 dots/cm$^2$.

2.  Method according to claim 1, **characterized in that** the spunbonded layer (A') formed in step (a) comprises continuous microfilaments having a diameter (DI) less than or equal to 10 $\mu$m.

3.  Method according to claim 1 or 2, **characterized in that** the pre-consolidation step (b) of the spunbonded layer (A') is performed with a bonding pattern having a bonding dots density (DD) that is higher than or equal to 100 dots/cm$^2$.

4.  Method according to any one of claims 1 to 3, **characterized in that** the pre-consolidation step (b) of the spunbonded layer (A') is performed with a bonding pattern having a low bonding ratio (R) less than 30%.

5.  Method according to claim 4, **characterized in that** the pre-consolidation step (b) of the spunbonded layer (A') is performed with a bonding pattern having a low bonding ratio (R) less than 20%.

6.  Method according to any one of claims 1 to 5, **characterized in that** the pre-consolidation step (b) of the spunbonded layer (A') is performed with a bonding pattern comprising bonding dots (210a) having a bonding area less than 0.5mm$^2$.

7.  Method according to claim 6, **characterized in that** the pre-consolidation step (b) of the spunbonded layer (A') is performed with a bonding pattern comprising bonding dots (210) having a bonding area less than 0.3mm$^2$.

8.  Method according to claim 7, **characterized in that** the pre-consolidation step (b) of the spunbonded layer (A') is performed with a bonding pattern comprising bonding dots (210) having a bonding area less than 0.2mm$^2$.

9.  Method according to any one of claims 1 to 8, **characterized in that** it further comprises an additional step of providing a nonwoven cover layer (C) onto and in contact with the absorbent pulp layer (B) before the step (d) of consolidating the composite nonwoven.

10. Method according to claim 9, **characterized in that** the nonwoven cover layer (C) is a carded layer.

**11.** Method according to claim 9, **characterized in that** the nonwoven cover layer (C) is a spunbonded layer.

**12.** Method according to claim 11, **characterized in that** the additional step of providing a nonwoven cover layer (C) comprises the following sub-steps :

(a') forming one spunbonded layer (C')
(b') thermo-bonding the said spunbonded layer (C'), in order to obtain a pre-consolidated spunbonded layer (C).

**13.** Method according to claim 12, **characterized in that** the spunbonded layer (C') formed in sub-step (a') comprises continuous microfilaments having a diameter (DI) less than or equal to 15 um, and **in that** the pre-consolidation sub-step (b') of the spunbonded layer (C') is performed by means of a bonding pattern having bonding dots (210a), and having technical characteristic defined in any one of claims 1, 3, 4, 5, 6, 7, 8 for the bonding pattern used in pre-consolidation step (b).

**14.** Method according to claim 12 or 13, **characterized in that** the spunbonded layer (C') formed in sub-step (a') comprises continuous microfilaments having a diameter (DI) less than or equal to 10 $\mu$m.

**15.** Method according to any one of claims 9 to 14, **characterized in that** the weight of the cover layer (C) is less than 30 g/m$^2$.

**16.** Method according to any one of claims 1 to 15, **characterized in that** the weight of the spunbonded layer (A') is less than 35 g/m$^2$, preferably less than 25 g/m$^2$, and more preferably less than 12 g/m$^2$.

**17.** Method according to any one of claims 1 to 16, **characterized in that** the weight of the pulp layer (B) is less than 50 g/m$^2$.

**18.** Method according to any one of claims 1 to 17, **characterized in that** the weight of the composite nonwoven is less than 115 g/m$^2$.

**19.** Method according to any one of claims 9 to 14, **characterized in that** the weight of the composite nonwoven (A/B/C) is between 27g/m$^2$ and 115 g/m$^2$, **in that** the weight of the pre-consolidated spunbonded carrier layer (A) is between 7 g/m$^2$ and 35 g/m$^2$, **in that** the weight of the pulp layer (B) is between 10 g/m$^2$ and 50 g/m$^2$, and **in that** the weight of the cover layer (C) is between 10 g/m$^2$ and 30 g/m$^2$.

**20.** An absorbent hydroentangled composite nonwoven comprising a pre-consolidated spunbonded layer (A) and an absorbent pulp layer (B) in contact with the pre-consolidated spunbonded layer (A), **characterized in that** the pre-consolidated spunbonded layer (A) comprises continuous microfilaments having a diameter (DI) less or equal to 15 $\mu$m, and bonded dots (210b), the density (DD) of said bonded dots (210b) being higher than or equal to 90 dots/cm$^2$.

**21.** Composite nonwoven according to claim 20, **characterized in that** the pre-consolidated spunbonded layer (A) comprises continuous microfilaments having a diameter (Di) less than or equal to 10 $\mu$m.

**22.** Composite nonwoven according to claim 20 or 21, **characterized in that** the density (DD) of the bonded dots (210b) of the pre-consolidated spunbonded layer (A) is higher than or equal to 100 dots/cm$^2$.

**23.** Composite nonwoven according to any one of claims of 20 to 22, **characterized in that** the bonding ratio (R') of the pre-consolidated spunbonded layer (A) is less than 30%.

**24.** Composite nonwoven according to claim 23. **characterized in that** the bonding ratio (R') of the pre-consolidated spunbonded layer (A) is less than 20%.

**25.** Composite nonwoven according to any one of claims 20 to 24, **characterized in that** bonded dots (210b) have an area less than 0.5mm$^2$.

**26.** Composite nonwoven according to claim 25, **characterized in that** bonded dots (210b) have an area less than 0.3mm$^2$.

**27.** Composite nonwoven according to claim 26, **characterized in that** bonded dots (210b) have an area less than

$0.2mm^2$.

28. Composite nonwoven according to any one of claims 20 to 27, **characterized in that** it further comprises an additional nonwoven cover layer (C) in contact with the absorbent pulp layer (B).

29. Composite nonwoven according to claim 28, **characterized in that** the nonwoven cover layer (C) is a carded layer.

30. Composite nonwoven according to claim 28, **characterized in that** the nonwoven cover layer (C) is a spunbonded layer.

31. Composite nonwoven according to claim 30, **characterized in that** the nonwoven cover layer (C) is a pre-consolidated spunbonded layer having technical characteristics defined in any one of claims. 20 to 27 for the other pre-consolidated spunbonded layer (A).

32. Composite nonwoven according to any one of claims 28 to 31, **characterized in that** the weight of the cover layer (C) is less than 30 $g/m^2$.

33. Composite nonwoven according to any one of claims 20 to 32. **characterized in that** the weight of the pre-consolidated spunbonded layer (A) is less than 35 $g/m^2$, preferably less than 25 $g/m^2$, and more preferably less than 12 $g/m^2$.

34. Composite nonwoven according to any one of claims 20 to 33, **characterized in that** the weight of the pulp layer (B) is less than 50 $g/m^2$.

35. Composite nonwoven according to any one of claims 20 to 34, **characterized in that** the weight of the composite nonwoven is less than 115 $g/m^2$.

36. Composite nonwoven according to any one of claims 28 to 31, **characterized in that** the weight of the composite nonwoven (A/B/C) is between 27$g/m^2$ and 115 $g/m^2$, **in that** the weight of the pre-consolidated spunbonded layer (A) is between 7 $g/m^2$ and 35 $g/m^2$, **in that in that** the weight of the pulp layer (B) is between 10 $g/m^2$ and 50 $g/m^2$, and **in that** the weight of the cover layer (C) is between 10 $g/m^2$ and 30 $g/m^2$.

37. Use of the composite nonwoven of any one of claims 20 to 36 for making hygienic products, and more particularly dry wipes, or wet wipes, or diapers, or training pants, or sanitary napkins, or incontinence products.

38. A continuous system for producing a hydroentangled absorbent composite nonwoven according to any one of claims 20 to 36, **characterized in that** it comprises :

   - a spunbonding unit (1) for producing a spunbonded layer (A') comprising continuous microfilaments having a diameter (DI) less than or equal to 15 $\mu$m,
   - a thermal bonding unit (2) mounted downstream the spunbonding unit (1) and comprising an engraved roll (21) having a bonding pattern **characterized by** a bonding dots density. (DD) that is higher than or equal to 90 dots/$cm^2$.
   - an air-laying unit (3), mounted downstream the spunbonding unit (1), and fed with pulp,
   - a hydraulic needling unit (5) mounted downstream the air-laying unit (3).

39. Continuous system according to claim 38, **characterized in that** the spunbonding unit (1) is set up in order to produce a spunbonded layer (A') comprising continuous microfilaments having a diameter (DI) less than or equal to 10 $\mu$m.

40. Continuous system according to claim 38 or 39, **characterized in that** the density of the bonding dots (210a) of the bonding pattern of engraved roll (21) is higher than or equal to 100 dots/$cm^2$.

41. Continuous system according to any one of claims 38 to 40, **characterized in that** the bonding ratio (R) of the bonding pattern of engraved roll (21) is less than 30%.

42. Continuous system according to claim 41, **characterized in that** the bonding ratio (R) of the bonding pattern of engraved roll (21) is less than 20%.

43. Continuous system according to any one of claims 38 to 42, **characterized in that** the bonding pattern of engraved roll (21) comprises bonding dots (210a) having an area less than 0.5mm$^2$.

44. Continuous system according to claim 43, **characterized in that** the bonding pattern of engraved roll (21) comprises bonding dots (210a) having an area less than 0.3mm$^2$.

45. Continuous system according to claim 44, **characterized in that** the bonding pattern of engraved roll (21) comprises bonding dots (210a) having an area less than 0.2mm$^2$.

46. Continuous system according to any one of claims 38 to 45. **characterized in that** it further comprises a carding unit (4) upstream the hydraulic needling unit (5)..

47. Continuous system according to any one of claims 38 to 45, **characterized in that** it further comprises an additional spunbonding unit (1') upstream the hydraulic needling unit (5).

48. continuous system according to claim 47, **characterized in that** the additional spunbonding unit (1') is set up in order to produce a spunbonded layer (C') comprising continuous microfilaments having a diameter (DI) less than or equal to 15$\mu$m, and preferably less than or equal to 10$\mu$m.

49. Continuous system according to claim 48, **characterized in that** it comprises an additional thermal bonding unit (2') that is mounted downstream the additional spunbonding unit (1') and that comprises an engraved roll (21'), and **in that** said engraved roll (21') has a bonding pattern having technical characteristics defined in any one of claims to 38, 40, 41, 42, 43, 44, 45 for the engraved roll (21) of the other thermal bonding unit (2).

**Patentansprüche**

1. Verfahren zum Herstellen eines hydroverhakten, saugfähigen Verbund-Faservlieses, wobei das Verfahren die folgenden Schritte aufweist:

   (a) Ausbilden einer Gespinstverbund-Schicht (A'),
   (b) thermisches Verfestigen der genannten Gespinstverbund-Schicht (A'), um eine vorverfestigte Gespinstverbund-Schicht (A)zu erhalten,
   (c) Ablegen einer saugfähigen Pulpe-Schicht (B) auf die und in Kontakt mit der vorverfestigten Gespinstverbund-Schicht (A),
   (d) Verfestigen des Verbund-Faservlieses durch hydrodynamisches Nadeln,

   **dadurch gekennzeichnet, dass** die in Schritt (a) gebildete Gespinstverbund-Schicht (A') Endlos-Mikrofilamente mit einem Durchmesser (DI) kleiner als oder gleich 15 $\mu$m aufweist und dass der Vorverfestigungs-Schritt (b) der Gespinstverbund-Schicht (A') mit Hilfe eines Verbindungsmusters mit Verbindungspunkten (210a) erfolgt, wobei die Dichte (DD) der Verbindungspunkte (210a) höher als oder gleich 90 Punkte/cm$^2$ ist.

2. Verfahren nach Anspruch 1, **dadurch gekennzeichnet, dass** die in Schritt (a) gebildete Gespinstverbund-Schicht (A') Endlos-Mikrofilamente mit einem Durchmesser (DI) von kleiner als oder gleich 10 $\mu$m aufweist.

3. Verfahren nach Anspruch 1 oder 2, **dadurch gekennzeichnet, dass** der Vorverfestigungs-Schritt (b) der Gespinstverbund-Schicht (A') mit einem Verbindungsmuster durchgeführt wird, das eine Dichte (DD) der Verbindungspunkte von mehr als oder gleich 100 Bunkten/cm$^2$ aufweist.

4. Verfahren nach einem der Ansprüche 1 bis 3, **dadurch gekennzeichnet, dass** der Vorverfestigungs-Schritt (b) der Gespinstverbund-Schicht (A') mit einem Verbindungsmuster durchgeführt wird, das ein niedriges Bindungsverhältnis (R) von weniger als 30% aufweist.

5. Verfahren nach Anspruch 4, **dadurch gekennzeichnet, dass** der Vorverfestigungs-Schritt (b) der Gespinstverbund-Schicht (A') mit einem Verbindungsmuster durchgeführt wird, das ein niedriges Bindungsverhältnis (R) von weniger als 20% aufweist.

6. Verfahren nach einem der Ansprüche 1 bis 5, **dadurch gekennzeichnet, dass** der Vorverfestigungs-Schritt (b) der Gespinstverbund-Schicht (A') mit einem Verbindungspunkte (210a) aufweisenden Verbindungsmuster durchgeführt wird, wobei die Verbindungspunkte eine Verbindungsfläche von weniger als 0,5 mm$^2$ aufweisen.

7.  Verfahren nach Anspruch 6, **dadurch gekennzeichnet, dass** der Vorverfestigungs-Schritt (b) der Gespinstverbund-Schicht (A') mit einem Verbindungspunkte (210a) aufweisenden Verbindungsmuster durchgeführt wird, wobei die Verbindungspunkte eine Verbindungsfläche von weniger als 0,3 mm$^2$ aufweisen.

8.  Verfahren nach Anspruch 7, **dadurch gekennzeichnet, dass** der Vorverfestigungs-Schritt (b) der Gespinstverbund-Schicht (A') mit einem Verbindungspunkte (210a) aufweisenden Verbindungsmuster durchgeführt wird, wobei die Verbindungspunkte eine Verbindungsfläche von weniger als 0,2 mm$^2$ aufweisen.

9.  Verfahren nach einem der Ansprüche 1 bis 8, **dadurch gekennzeichnet, dass** es weiterhin einen zusätzlichen Schritt des Bereitstellens einer Faservlies-Deckschicht (C) auf und in Kontakt mit der saugfähigen Pulpe-Schicht (B) vor dem Schritt (d) des Verfestigens des Verbund-Faservlieses umfasst.

10. Verfahren nach Anspruch 9, **dadurch gekennzeichnet, dass** die Faservlies-Deckschicht (C) eine kardierte Schicht ist.

11. Verfahren nach Anspruch 9, **dadurch gekennzeichnet, dass** die Faservlies-Deckschicht (C) eine Gespinstverbund-Schicht ist.

12. Verfahren nach Anspruch 11, **dadurch gekennzeichnet, dass** der zusätzliche Schritt des Bereitstellens einer Faservlies-Deckschicht (C) die folgenden Teilschritte umfasst:

    (a') Bilden einer Gespinstverbund-Schicht (C'),
    (b') thermisches Verfestigen der genannten Gespinstverbund-Schicht (C'), um eine vorverfestigte Gespinstverbund-Schicht (C) zu erhalten.

13. Verfahren nach Anspruch 12, **dadurch gekennzeichnet, dass** die in Teilschritt (a') gebildete Gespinstverbund-Schicht (C') Endlos-Mikrofilamente mit einem Durchmesser (DI) von kleiner als oder gleich 15 $\mu$m aufweist, und dass der Vorverfestigungs-Teilschritt (b') der Gespinstverbund-Schicht (C') mit Hilfe eines Verbindungsmusters mit Verbindungspunkten (210a) durchgeführt wird und technische Eigenschaften aufweist, wie sie in einem der Ansprüche 1, 3, 4, 5, 6, 7, 8 für das im Vorverfestigungs-Schritt (b) eingesetzte Verbindungsmuster definiert sind.

14. Verfahren nach Anspruch 12 oder 13, **dadurch gekennzeichnet, dass** die im Teilschritt (a') gebildete Gespinstverbund-Schicht (C') Endlos-Mikrofilamente mit einem Durchmesser (DI) von weniger als oder gleich 10 $\mu$m aufweist.

15. Verfahren nach einem der Ansprüche 9 bis 14, **dadurch gekennzeichnet, dass** das Gewicht der Deckschicht (C) unter 30 g/m$^2$ liegt.

16. Verfahren nach einem der Ansprüche 1 bis 15, **dadurch gekennzeichnet, dass** das Gewicht der Gespinstverbund-Schicht (A') unter 35 g/m$^2$, vorzugsweise unter 25 g/m$^2$ und stärker bevorzugt unter 12 g/m$^2$ liegt.

17. Verfahren nach einem der Ansprüche 1 bis 16, **dadurch gekennzeichnet, dass** das Gewicht der Pulpe-Schicht (B) unter 50 g/m$^2$ liegt.

18. Verfahren nach einem der Ansprüche 1 bis 17, **dadurch gekennzeichnet, dass** das Gewicht des Verbund-Faservlieses unter 115 g/m$^2$ liegt.

19. Verfahren nach einem der Ansprüche 9 bis 14, **dadurch gekennzeichnet, dass** das Gewicht des Verbund-Faservlieses (A/B/C) zwischen 27 g/m$^2$ und 115 g/m$^2$ liegt, dass das Gewicht der vorverfestigten Gespinstverbund-Trägerschicht (A) zwischen 7 g/m$^2$ und 35 g/m$^2$ liegt, dass das Gewicht der Pulpe-Schicht (B) zwischen 10 g/m$^2$ und 50 g/m$^2$ liegt, und dass das Gewicht der Deckschicht (C) zwischen 10 g/m$^2$ und 30 g/m$^2$ liegt.

20. Saugfähiges, hydroverhaktes Verbund-Faservlies, umfassend eine vorverfestigte Gespinstverbund-Schicht (A) und eine saugfähige Pulpe-Schicht (B) in Kontakt mit der vorverfestigten Gespinstverbund-Schicht (A), **dadurch gekennzeichnet, dass** die vorverfestigte Gespinstverbund-Schicht (A) Endlos-Mikrofilamente mit einem Durchmesser (DI) von weniger als oder gleich 15 $\mu$m sowie Bindungspunkte (210b) aufweist, wobei die Dichte (DD) der genannten Bindungspunkte (210b) höher als oder gleich 90 Punkte/cm$^2$ ist.

21. Verbund-Faservlies nach Anspruch 20, **dadurch gekennzeichnet, dass** die vorverfestigte Gespinstverbund-Schicht (A) Endlos-Mikrofilamente mit einem Durchmesser (DI) von weniger als oder gleich 10 μm aufweist.

22. Verbund-Faservlies nach Anspruch 20 oder 21, **dadurch gekennzeichnet, dass** die Dichte (DD) der Bindungspunkte (210b) der vorverfiestigten Gespinstverbund-Schicht (A) größer als oder gleich 100 Punkte/cm$^2$ ist.

23. Verbund-Faservlies nach einem der Ansprüche 20 bis 22, **dadurch gekennzeichnet, dass** das Bindungsverhältnis (R') der vorverfestigten Gespinstverbund-Schicht (A) unter 30% liegt.

24. Verbund-Faservlies nach Anspruch 23, **dadurch gekennzeichnet, dass** das Bindungsverhältnis (R') der vorverfestigten Gespinstverbund-Schicht (A) unter 20% liegt.

25. Verbund-Faservlies nach einem der Ansprüche 20 bis 24, **dadurch gekennzeichnet, dass** Bindungspunkte (210b) eine Fläche von unter 0,5 mm$^2$ aufweisen.

26. Verbund-Faservlies nach Anspruch 25, **dadurch gekennzeichnet, dass** Bindungspunkte (210b) eine Fläche von unter 0,3 mm$^2$ aufweisen.

27. Verbund-Faservlies nach Anspruch 26, **dadurch gekennzeichnet, dass** Bindungspunkte (210b) eine Fläche von unter 0,2 mm$^2$ aufweisen.

28. Verbund-Faservlies nach einem der Ansprüche 20 bis 27, **dadurch gekennzeichnet, dass** es weiterhin eine zusätzliche Faservlies-Deckschicht (C) in Kontakt mit der saugfähigen Pulpe-Schicht (B) aufweist.

29. Verbund-Faservlies nach Anspruch 28, **dadurch gekennzeichnet, dass** die Faservlies-Deckschicht in (C) eine kardierte Schicht ist.

30. Verbund-Faservlies nach Anspruch 28, **dadurch gekennzeichnet, dass** die Faservlies-Deckschicht (C) eine Gespinstverbund-Schicht ist.

31. Verbund-Faservlies nach Anspruch 30, **dadurch gekennzeichnet, dass** die Faservlies-Deckschicht (C) eine vorverfestigte Gespinstverbund-Schicht mit technischen Eigenschaften ist, die in einem der Ansprüche 20 bis 27 für die andere vorverfestigte Gespinstverbund-Schicht (A) definiert sind.

32. Verbund-Faservlies nach einem der Ansprüche 28 bis 31, **dadurch gekennzeichnet, dass** das Gewicht der Deckschicht (C) unter 30 g/m$^2$ beträgt.

33. Verbund-Faservlies nach einem der Ansprüche 20 bis 32, **dadurch gekennzeichnet, dass** das Gewicht der vorverfestigten Gespinstverbund-Schicht (A) unter 35 g/m$^2$, vorzugsweise unter 25 g/m$^2$ und stärker bevorzugt unter 12 g/m$^2$ liegt.

34. Verbund-Faservlies nach einem der Ansprüche 20 bis 33, **dadurch gekennzeichnet, dass** das Gewicht der Pulpe-Schicht (B) unter 50 g/m$^2$ liegt.

35. Verbund-Faservlies nach einem der Ansprüche 20 bis 34, **dadurch gekennzeichnet, dass** das Gewicht des Ver-bundFaservlieses unter 115 g/m$^2$ liegt.

36. Verbund-Faservlies nach einem der Ansprüche 28 bis 31, **dadurch gekennzeichnet, dass** das Gewicht des Verbund-Faservlieses (A/B/C) zwischen 27 g/m$^2$ und 115 g/m$^2$ liegt, dass das Gewicht der vorverfestigten Gespinstverbund-Schicht (A) zwischen 7 g/m$^2$ und 35 g/m$^2$ liegt, dass das Gewicht der Pulpe-Schicht (B) zwischen 10 g/m$^2$ und 50 g/m$^2$ liegt, und dass das Gewicht der Deckschicht (C) zwischen 10 g/m$^2$ und 30 g/m$^2$ liegt.

37. Verwendung des Verbund-Faservlieses nach einem der Ansprüche 20 bis 36 zur Herstellung von Hygieneprodukten und insbesondere von trockenen Wischtüchern oder feuchten Wischtüchern oder Windeln oder Trainings-Hosen oder Damenbinden oder Inkontinzenz-Produkten.

38. Ein kontinuierliches System zur Erstellung eines hydroverhakten saugfähigen Verbund-Faservlieses nach einem der Ansprüche 20 bis 36, **dadurch gekennzeichnet, dass** es umfasst:

- eine Spinnvlies-Einheit (1) zum Herstellen einer Gespinstverbund-Schicht (A') mit Endlos-Mikrofilamenten, die einen Durchmesser (DI) von kleiner als oder gleich 15 $\mu$m aufweisen,
- eine Einheit (2) zum thermischen Verfestigen, hinter der Spinnvlies-Einrichtung (1) angeordnet und eine Gravurwalze (21) mit einem Verbindungsmuster umfassend, das durch eine Dichte (DD) der Verbindungspunkte von mehr als oder gleich 90 Punkten/cm$^2$ **gekennzeichnet** ist,
- eine Luftstrom-Einheit (3), hinter der Spinnvlies-Einheit (1) angeordnet und mit Pulpe beaufschlagt,
- eine Einheit (5) zum hydraulischen Nadeln, hinter der Luftstrom-Einheit (3) gelegen.

39. Kontinuierliches System nach Anspruch 38, **dadurch gekennzeichnet, dass** die Spinnvlies-Einheit (1) so eingerichtet ist, dass sie eine Gespinstverbund-Schicht (A') erzeugen kann, die Endlos-Mikrofilamente mit einem Durchmesser (DI) von weniger als oder gleich 10$\mu$m aufweist.

40. Kontinuierliches System nach Anspruch 38 oder 39, **dadurch gekennzeichnet, dass** die Dichte der Verbindungspunkte (210a) des Verbindungsmusters der Gravurwalze (21) höher als oder gleich 100 Punkte/cm$^2$ ist.

41. Kontinuierliches System nach einem der Ansprüche 38 bis 40, **dadurch gekennzeichnet, dass** das Bindungsverhältnis (R) des Verbindungsmusters der Gravurwalze (21) unter 30% liegt.

42. Kontinuierliches System nach Anspruch 41, **dadurch gekennzeichnet, dass** das Bindungsverhältnis (R) des Verbindungsmusters der Gravurwalze (21) unter 20% liegt.

43. Kontinuierliches System nach einem der Ansprüche 38 bis 42, **dadurch gekennzeichnet, dass** das Verbindungsmuster der Gravurwalze (21) Verbindungspunkte (210a) mit einer Fläche von unter 0,5 mm$^2$ aufweist.

44. Kontinuierliches System nach Anspruch 43, **dadurch gekennzeichnet, dass** das Verbindungsmuster der Gravurwalze (21) Verbindungspunkte (210a) mit einer Fläche von unter 0,3 mm$^2$ aufweist.

45. Kontinuierliches System nach Anspruch 44, **dadurch gekennzeichnet, dass** das Verbindungsmuster der Gravurwalze (21) Verbindungspunkte (210a) mit einer Fläche von unter 0,2 mm$^2$ aufweist.

46. Kontinuierliches System nach einem der Ansprüche 38 bis 45, **dadurch gekennzeichnet, dass** es weiterhin eine Kardiereinheit (4) vor der Einheit für das hydraulische Nadeln (5) aufweist.

47. Kontinuierliches System nach einem der Ansprüche 38 bis 45, **dadurch gekennzeichnet, dass** es weiterhin eine zusätzliche Spinnvlies-Einheit (1') vor der Einheit für das hydraulische Nadeln (5) aufweist.

48. Kontinuierliches System nach Anspruch 47, **dadurch gekennzeichnet, dass** die zusätzliche Spinnvlies-Einheit (1') so eingerichtet ist, dass sie eine Gespinstverbund-Schicht (C') mit Endlos-Mikrofilamenten erzeugen kann, die einen Durchmesser (DI) von kleiner als oder gleich 15 $\mu$m und vorzugsweise von kleiner als oder gleich 10 $\mu$m besitzen.

49. Kontinuierliches System nach Anspruch 48, **dadurch gekennzeichnet, dass** es eine zusätzliche Einrichtung zum thermischen Verfestigen (2') aufweist, die hinter der zusätzlichen Spinnvlies-Einheit (1') angeordnet ist, und dass es eine Gravurwalze (21') aufweist, und dass die genannte Gravurwalze (21') ein Verbindungsmuster mit technischen Eigenschaften besitzt, die in einem der Ansprüche 38, 40, 41, 42, 43, 44, 45 für die Gravurwalze (21) der anderen Einheit zum thermischen Verfestigen (2) definiert sind.

**Revendications**

1. Procédé de fabrication d'un non-tissé composite absorbant hydroenchevêtré, ledit procédé comprenant les étapes suivantes :

(a) former une couche de filé-lié (A'),
(b) thermolier ladite couche de filé-lié (A'), afin d'obtenir une couche de filé-lié pré-consolidée (A),

(c) déposer une couche de pâte absorbante (B) sur et en contact avec la couche de filé-lié pré-consolidée (A),

(d) consolider le non-tissé composite par aiguilletage hydrodynamique,

**caractérisé en ce que** la couche de filé-lié (A') formée à l'étape (a) comprend des microfilaments continus ayant un diamètre (DI) inférieur ou égal à 15 μm, et **en ce que** l'étape de pré-consolidation (b) de la couche de filé-lié (A') est effectuée au moyen d'un motif de liage ayant des points de liage (210a), la densité (DD) desdits points de liage (210a) étant supérieure ou égale à 90 points/cm$^2$.

**2.** Procédé selon la revendication 1, **caractérisé en ce que** la couche de filé-lié (A') formée à l'étape (a) comprend des microfilaments continus ayant un diamètre (DI) inférieur ou égal à 10 μm.

**3.** Procédé selon la revendication 1 ou 2, **caractérisé en ce que** l'étape de pré-consolidation (b) de la couche de filé-lié (A') est effectuée avec un motif de liage ayant une densité de points de liage (DD) qui est supérieure ou égale à 100 points/cm$^2$.

**4.** Procédé selon l'une quelconque des revendications 1 à 3, **caractérisé en ce que** l'étape de pré-consolidation (b) de la couche de filé-lié (A') est effectuée avec un motif de liage ayant un faible taux de liage (R) inférieur à 30 %.

**5.** Procédé selon la revendication 4, **caractérisé en ce que** l'étape de pré-consolidation (b) de la couche de filé-lié (A') est effectuée avec un motif de liage ayant un faible taux de liage (R) inférieur à 20 %.

**6.** Procédé selon l'une quelconque des revendications 1 à 5, **caractérisé en ce que** l'étape de pré-consolidation (b) de la couche de filé-lié (A') est effectuée avec un motif de liage comprenant des points de liage (210a) ayant une surface de liage inférieure à 0,5 mm$^2$.

**7.** Procédé selon la revendication 6, **caractérisé en ce que** l'étape de pré-consolidation (b) de la couche de filé-lié (A') est effectuée avec un motif de liage comprenant des points de liage (210) ayant une surface de liage inférieure à 0,3 mm$^2$.

**8.** Procédé selon la revendication 7, **caractérisé en ce que** l'étape de pré-consolidation (b) de la couche de filé-lié (A') est effectuée avec un motif de liage comprenant des points de liage (210) ayant une surface de liage inférieure à 0,2 mm$^2$.

**9.** Procédé selon l'une quelconque des revendications 1 à 8, **caractérisé en ce qu'**il comprend en outre une étape supplémentaire d'application d'une couche de couverture de non-tissé (C) sur et en contact avec la couche de pâte absorbante (B) avant l'étape (d) de consolidation du non-tissé composite.

**10.** Procédé selon la revendication 9, **caractérisé en ce que** la couche de couverture de non-tissé (C) est une couche cardée.

**11.** Procédé selon la revendication 9, **caractérisé en ce que** la couche de couverture de non-tissé (C) est une couche de filé-lié.

**12.** Procédé selon la revendication 11, **caractérisé en ce que** l'étape supplémentaire d'application d'une couche de couverture de non-tissé (C) comprend les sous-étapes suivantes :

(a') formation d'une couche de filé-lié (C')

(b') thermoliage de ladite couche de filé-lié (C'), afin d'obtenir une couche de filé-lié pré-consolidée (C).

**13.** Procédé selon la revendication 12, **caractérisé en ce que** la couche de filé-lié (C') formée à la sous-étape (a') comprend des microfilaments continus ayant un diamètre (DI) inférieur ou égal à 15 μm, et **en ce que** la sous-étape de pré-consolidation (b') de la couche de filé-lié (C) est effectuée au moyen d'un motif de liage ayant des points de liage (210a), et ayant les caractéristiques techniques définies dans l'une quelconque des revendications 1, 3, 4, 5, 6, 7, 8 pour le motif de liage utilisé dans l'étape de pré-consolidation (b).

**14.** Procédé selon la revendication 12 ou 13, **caractérisé en ce que** la couche de filé-lié (C') formée à la sous-étape (a') comprend des microfilaments continus ayant un diamètre (DI) inférieur ou égal à 10 μm.

15. Procédé selon l'une quelconque des revendications 9 à 14, **caractérisé en ce que** le poids de la couche de couverture (C) est inférieur à 30 g/m$^2$.

16. Procédé selon l'une quelconque des revendications 1 à 15, **caractérisé en ce que** le poids de la couche de filé-lié (A') est inférieur à 35 g/m$^2$, de préférence inférieur à 25 g/m$^2$, et plus préférablement inférieur à 12 g/m$^2$.

17. Procédé selon l'une quelconque des revendications 1 à 16, **caractérisé en ce que** le poids de la couche de pâte (B) est inférieur à 50 g/m$^2$.

18. Procédé selon l'une quelconque des revendications 1 à 17, **caractérisé en ce que** le poids du non-tissé composite est inférieur à 115 g/m$^2$.

19. Procédé selon l'une quelconque des revendications 9 à 14, **caractérisé en ce que** le poids du non-tissé composite (A/B/C) est compris entre 27 g/m$^2$ et 115 g/m$^2$, **en ce que** le poids de la couche de support de filé-lié pré-consolidée (A) est compris entre 7 g/m$^2$ et 35 g/m$^2$, **en ce que** le poids de la couche de pâte (B) est compris entre 10 g/m$^2$ et 50 g/m$^2$, et **en ce que** le poids de la couche de couverture (C) est compris entre 10 g/m$^2$ et 30 g/m$^2$.

20. Non-tissé composite hydroenchevêtré absorbant comprenant une couche de filé-lié pré-consolidée (A) et une couche couche de pâte absorbante (B) en contact avec la couche de filé-lié pré-consolidée (A), **caractérisé en ce que** la couche de filé-lié pré-consolidée (A) comprend des microfilaments continus ayant un diamètre (DI) inférieur ou égal à 15 $\mu$m, et des points liés (210b), la densité (DD) desdits points liés (210b) étant supérieure ou égale à 90 points/cm$^2$.

21. Non-tissé composite selon la revendication 20, **caractérisé en ce que** la couche de filé-lié pré-consolidée (A) comprend des microfilaments continus ayant un diamètre (DI) inférieur ou égal à 10 $\mu$m.

22. Non-tissé composite selon la revendication 20 ou 21, **caractérisé en ce que** la densité (DD) des points liés (210b) de la couche de filé-lié pré-consolidée (A) est supérieure ou égale à 100 points/cm$^2$.

23. Non-tissé composite selon l'une quelconque des revendications 20 à 22, **caractérisé en ce que** le taux de liage (R') de la couche de filé-lié pré-consolidée (A) est inférieur à 30 %.

24. Non-tissé composite selon la revendication 23, **caractérisé en ce que** le taux de liage (R') de la couche de filé-lié pré-consolidée (A) est inférieur à 20 %.

25. Non-tissé composite selon l'une quelconque des revendications 20 à 24, **caractérisé en ce que** les points liés (210b) ont une surface inférieure à 0,5 mm$^2$.

26. Non-tissé composite selon la revendication 25, **caractérisé en ce que** les points liés (210b) ont une surface inférieure à 0,3 mm$^2$.

27. Non-tissé composite selon la revendication 26, **caractérisé en ce que** les points liés (210b) ont une surface inférieure à 0,2 mm$^2$.

28. Non-tissé composite selon l'une quelconque des revendications 20 à 27, **caractérisé en ce qu'**il comprend en outre une couche de couverture de non-tissé supplémentaire (C) en contact avec la couche de pâte absorbante (B).

29. Non-tissé composite selon la revendication 28, **caractérisé en ce que** la couche de couverture de non-tissé (C) est une couche cardée.

30. Non-tissé composite selon la revendication 28, **caractérisé en ce que** la couche de couverture de non-tissé (C) est une couche de filé-lié.

31. Non-tissé composite selon la revendication 30, **caractérisé en ce que** la couche de couverture de non-tissé (C) est une couche de filé-lié pré-consolidée ayant les caractéristiques techniques définies dans l'une quelconque des revendications 20 à 27 pour la couche de filé-lié pré-consolidée (A).

32. Non-tissé composite selon l'une quelconque des revendications 28 à 31, **caractérisé en ce que** le poids de la couche de couverture (C) est inférieur à 30 g/m$^2$.

33. Non-tissé composite selon l'une quelconque des revendications 20 à 32, **caractérisé en ce que** le poids de la couche de filé-lié pré-consolidée (A) est inférieur à 35 g/m$^2$, de préférence inférieur à 25 g/m$^2$, et plus préférablement inférieur à 12 g/m$^2$.

34. Non-tissé composite selon l'une quelconque des revendications 20 à 33, **caractérisé en ce que** le poids de la couche de pâte (B) est inférieur à 50 g/m$^2$.

35. Non-tissé composite selon l'une quelconque des revendications 20 à 34, **caractérisé en ce que** le poids du non-tissé composite est inférieur à 115 g/m$^2$.

36. Non-tissé composite selon l'une quelconque des revendications 28 à 31, **caractérisé en ce que** le poids du non-tissé composite (A/B/C) est compris entre 27 g/m$^2$ et 115 g/m$^2$, **en ce que** le poids de la couche de filé-lié pré-consolidée (A) est compris entre 7 g/m$^2$ et 35 g/m$^2$, **en ce que** le poids de la couche de pâte (B) est compris entre 10 g/m$^2$ et 50 g/m$^2$, et **en ce que** le poids de la couche de couverture (C) est compris entre 10 g/m$^2$ et 30 g/m$^2$.

37. Utilisation du non-tissé composite selon l'une quelconque des revendications 20 à 36 pour fabriquer des produits hygiéniques, et plus particulièrement des lingettes sèches, ou des lingettes humides, ou des couches, ou des culottes de propreté, ou des serviettes hygiéniques ou des produits pour l'incontinence.

38. Système continu pour fabriquer un non-tissé composite absorbant hydroenchevêtré selon l'une quelconque des revendications 20 à 36, **caractérisé en ce qu'**il comprend :

   - une unité de filage-liage (1) pour produire une couche de filé-lié (A') comprenant des microfilaments continus ayant un diamètre (DI) inférieur ou égal à 15 μm,
   - une unité de thermoliage (2) montée en aval de l'unité de filage-liage (1) et comprenant un rouleau gravé (21) ayant un motif de liage **caractérisé par** une densité de points de liage (DD) qui est supérieure ou égale à 90 points/cm$^2$,
   - une unité de dépose par voie sèche (3) montée en aval de l'unité de filage-liage (1) et alimentée avec de la pâte,
   - une unité d'aiguilletage hydraulique (5) montée en aval de l'unité de dépose par voie sèche (3).

39. Système continu selon la revendication 38, **caractérisé en ce que** l'unité de filage-liage (1) est configurée afin de produire une couche de filé-lié (A') comprenant des microfilaments continus ayant un diamètre (DI) inférieur ou égal à 10 μm.

40. Système continu selon la revendication 38 ou 39, **caractérisé en ce que** la densité des points de liage (210a) du motif de liage du rouleau gravé (21) est supérieure ou égale à 100 points/cm$^2$.

41. Système continu selon l'une quelconque des revendications 38 à 40, **caractérisé en ce que** le taux de liage (R) du motif de liage du rouleau gravé (21) est inférieur à 30 %.

42. Système continu selon la revendication 41, **caractérisé en ce que** le taux de liage (R) du motif de liage du rouleau gravé (21) est inférieur à 20 %.

43. Système continu selon l'une quelconque des revendications 38 à 42, **caractérisé en ce que** le motif de liage du rouleau gravé (21) comprend des points de liage (210a) ayant une surface inférieure à 0,5 mm$^2$.

44. Système continu selon la revendication 43, **caractérisé en ce que** le motif de liage du rouleau gravé (21) comprend des points de liage (210a) ayant une surface inférieure à 0,3 mm$^2$.

45. Système continu selon la revendication 44, **caractérisé en ce que** le motif de liage du rouleau gravé (21) comprend des points de liage (210a) ayant une surface inférieure à 0,2 mm$^2$.

46. Système continu selon l'une quelconque des revendications 38 à 45, **caractérisé en ce qu'**il comprend en outre une unité de cardage (4) en amont de l'unité d'aiguilletage hydraulique (5).

47. Système continu selon l'une quelconque des revendications 38 à 45, **caractérisé en ce qu'**il comprend en outre une unité de filage-liage supplémentaire (1') en amont de l'unité d'aiguilletage hydraulique (5).

**48.** Système continu selon la revendication 47, **caractérisé en ce que** l'unité de filage-liage supplémentaire (1') est configurée afin de produire une couche de filé-lié (C') comprenant des microfilaments continus ayant un diamètre (DI) inférieur ou égal à 15 μm, et de préférence inférieur ou égal à 10 μm.

**49.** Système continu selon la revendication 48, **caractérisé en ce qu'**il comprend une unité de thermoliage supplémentaire (2') qui est montée en aval de l'unité de filage-liage supplémentaire (1') et qui comprend un rouleau gravé (21'), et **en ce que** ledit rouleau gravé (21') a un motif de liage ayant les caractéristiques techniques définies dans l'une quelconque des revendications 38, 40, 41, 42, 43, 44, 45 pour le rouleau gravé (21) de l'autre unité de thermoliage (2).

Fig.1

Fig.2

EP 1 961 850 B1

Fig.3

Fig.4

L2

L1

MD ↑

CD →

Fig.5

VII

VI

VII

VI

210

D1

210a

D2

L1

210a

210

H

Fig.6

D1

D2

210a

L2

210

H

Fig.7

MD ↑

CD →

Fig.8

Fig.9

210b

Fig.10

210b

Fig.11

P

Fig.12A

P

Fig.12B

P

Fig.12C

P P'

Fig.12D

P' P

Fig.12E

P P'

Fig.12F

P P'

Fig.12G

P P'

Fig.12H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0540041 A
- US 3560328 A
- WO 92080834 A
- US 6836937 B
- WO 2004092472 A
- WO 2006010766 A
- WO 0153588 A
- US 6836938 B
- US 20030207636 A
- WO 0153590 A
- EP 0032772 A